# EUROPEAN PATENT SPECIFICATION

(11) **EP 2 732 750 B1**
(45) Date of publication and mention of the grant of the patent: **10.05.2017**
(21) Application number: 12810608.5
(22) Date of filing: 10.07.2012
(51) Int. Cl.: A61B 1/00, G02B 23/24, A61B 1/005

(54) **ENDOSCOPE**
ENDOSKOP
ENDOSCOPE

(30) Priority: 11.07.2011 JP 2011153208
(43) Date of publication of application: 21.05.2014
(73) Proprietor: Olympus Corporation, Tokyo 192-8507 (JP)
(72) Inventor: MIYOSHI, Hiroaki, Hachioji-shi, Tokyo 192-8507 (JP); ISHIZAKI, Ryosuke, Hachioji-shi, Tokyo 192-8507 (JP)
(74) Representative: Schicker, Silvia
(86) International application number: PCT/JP2012/067604
(87) International publication number: WO 2013/008821

(56) References cited:
- EP-A1- 0 301 288
- EP-A1- 2 103 247
- JP-A- 60 048 725
- JP-A- 2004 154 177
- JP-A- 2005 046 279
- US-A- 5 179 935
- US-A1- 2010 280 449

## Description

### Technical Field

The present invention relates to an endoscope including a plurality of bending portions.

### Background Art

For example, Jpn. Pat. Appln. KOKAI Publication No. 2006-218231 discloses an endoscope including a first bending portion (an active bending portion) that bends in accordance with an operation of a bending operation knob in an operation section and a second bending portion (a passive bending portion) that is arranged at a proximal end portion of the first bending portion and passively bends by external force.

For example, in case of inserting an insertion section of the endoscope disclosed in Jpn. Pat. Appln. KOKAI Publication No. 2006-218231 into a lumen such as a large intestine, the first bending portion is bent, and the insertion section is pushed into the large intestine while capturing a depth direction (an insertion enabling direction) in an endoscopic display. For example, when an inner wall surface of the large intestine applies the external force to the second bending portion and a bending direction of the second bending portion is opposite to a bending direction of the first bending portion, pushing in the insertion section toward the inner side of the larger intestine may be difficult since the second bending portion bends in some cases.

Further, in a case where the external force is applied to the second bending portion and the second bending portion bends in the opposite direction of the bending direction of the first bending portion when the first bending portion is bent and the insertion section is pulled toward the front side while catching a distal end of the insertion section on the inner wall of the large intestine with to hold the large intestine, bending of the second bending portion may cancel the state that the large intestine is held by the first bending portion in some cases.

Therefore, there has been demanded an endoscope in which a second bending portion hardly bends in a direction opposite to a bending direction of a first bending portion when the first bending portion is bent in a given direction.

Finally, document EP 0 301 288 A1 discloses an endoscope according to the preamble of claim 1. Further relevant prior art is found in documents US 2010/280449 A1, EP 2 103 247 A1 and US 5,179,935 A.

### Summary of Invention

It is an object of the present invention to provide an endoscope in which a second bending portion hardly bends in a direction opposite to a bending direction of a first bending portion when the first bending portion is bent from a straight state.

This object is solved by an endoscope according to claim 1. Preferable embodiments are set-forth in the dependent claims.

### Brief Description of Drawings

FIG. 1A is a schematic view showing an endoscope according to each of first to fourth embodiments;
FIG. 1B is a schematic perspective view showing a part of a bend tube of a first bending portion in an insertion section of the endoscope according to each of the first to fourth embodiments;
FIG. 2A is a schematic longitudinal cross-sectional view showing an internal configuration of the insertion section and an operation section of the endoscope according to the first embodiment;
FIG. 2B is a schematic longitudinal cross-sectional view showing the internal configuration of the insertion section and the operation section of the endoscope according to the first embodiment;
FIG. 2C is a schematic transverse cross-sectional view taken along a line 2C-2C in each of FIG. 2A and FIG. 2B;
FIG. 3A is a schematic longitudinal cross-sectional view showing an internal configuration of an insertion section and an operation section of an endoscope according to a modification of the first embodiment;
FIG. 3B is a schematic longitudinal cross-sectional view showing the internal configuration of the insertion section and the operation section of the endoscope according to the modification of the first embodiment;

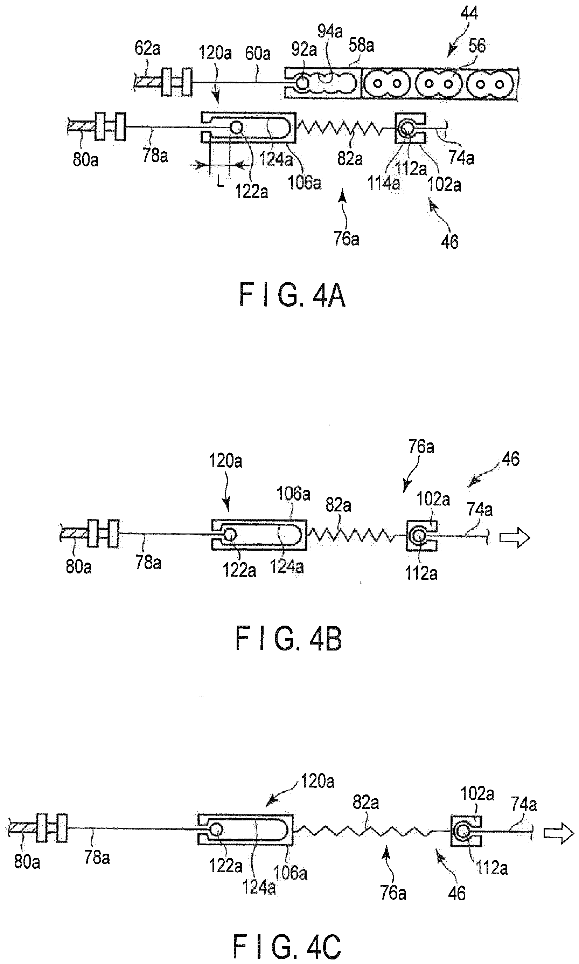

FIG. 4A is a schematic view showing an outline configuration of a first bending drive mechanism and a second bending drive mechanism near a boundary between an insertion section and an operation section of an endoscope according to a second embodiment, and also showing that an engagement convex portion at a proximal end of an angle wire is engaged with an engagement concave portion of a connection member at an end portion of a chain that interlocks with rotational movement of a first sprocket of the first bending drive mechanism and that a slider of a distal end-side traction member of the second bending drive mechanism can freely move in the range of a slider receiving potion of a distal end-side coupling member;
FIG. 4B is a schematic view showing that a proximal end-side traction member of the second bending drive mechanism near the boundary between the insertion section and the operation section of the endoscope according to the second embodiment is pulled, the distal end-side member of the same moves, and the slider of the distal end-side traction member thereby comes into contact with a distal end of the slider receiving portion of the distal end-side coupling member;
FIG. 4C is a schematic view showing that the proximal end-side traction member of the second bending drive mechanism near the boundary between the insertion section and the operation section of the endoscope according to the second embodiment is further pulled from the state depicted in FIG. 4B, an elastic member is stretched, the distal end-side member moves, and the slider of the distal end-side traction member thereby comes into contact with the distal end of the slider receiving member of the distal end-side coupling member and pulls the distal end-side traction member;
FIG. 5 is a schematic view showing an outline configuration of one of a pair of buffer portions in a second bending drive mechanism near a boundary between an insertion section and an operation section of an endoscope according to a third embodiment;
FIG. 6 is a schematic view showing an outline configuration of one of a pair of buffer portions in a second bending drive mechanism near a boundary between an insertion section and an operation section of an endoscope according to a fourth embodiment;
FIG. 7 is a schematic view showing an outline configuration of the pair of buffer portions of the second bending drive mechanism near the boundary between the insertion section and the operation section of the endoscope according to the fourth embodiment;
FIG. 8 is a schematic longitudinal cross-sectional view showing a buffer member having a second sprocket and a drum arranged on a basal plate in an operation section of an endoscope according to a fifth embodiment;
FIG. 9A is a schematic longitudinal cross-sectional view showing an internal configuration of an insertion section and the operation section of the endoscope according to the fifth embodiment in a state that a first bending portion is straight;
FIG. 9B is a schematic front view showing the drum which is arranged in the operation section of the endoscope according to the fifth embodiment and supported to allow its rotational movement with respect to the second sprocket;
FIG. 10A is a schematic longitudinal cross-sectional view showing the internal configuration of the insertion section and the operation section of the endoscope according to the fifth embodiment in a state that the first bending portion is bent in a U direction at approximately 90 degrees;
FIG. 10B is a schematic front view showing a drum that is arranged in the operation section of the endoscope according to the fifth embodiment and is supported to rotationally move with respect to the second sprocket when the second sprocket rotationally moves approximately 90 degrees from the state depicted in each of FIG. 9A and FIG. 9B;
FIG. 11A is a schematic longitudinal cross-sectional view showing the internal configuration of the insertion section and the operation section of the endoscope according to the fifth embodiment in a state that the first bending portion is bent in the U direction at approximately 180 degrees;
FIG. 11B is a schematic front view showing the drum that is arranged in the operation section of the endoscope according to the fifth embodiment and rotationally moved by the second sprocket when the second sprocket rotationally moves substantially 180 degrees from the state depicted in each of FIG. 9A and FIG. 9B and or substantially 90 degrees from the state depicted in each of FIG. 10A and FIG. 10B;
FIG. 12A is a schematic front view showing a drum that is arranged in an operation section of an endoscope according to a modification of the fifth embodiment and supported to be rotationally movable with respect to a second sprocket;
FIG. 12B is a schematic front view showing a drum that is arranged in an operation section of an endoscope according to a modification of the fifth embodiment and supported to be rotationally movable with respect to a second sprocket;
FIG. 13A is a schematic view showing an endoscope according to a sixth embodiment;
FIG. 13B is a schematic perspective view showing part of a bending tube of a first bending portion of an insertion section in the endoscope according to the sixth embodiment;
FIG. 14A is a schematic longitudinal cross-sectional view showing an internal configuration of the insertion section and an operation section of the endoscope according to the sixth embodiment;
FIG. 14B is a schematic longitudinal cross-sectional view showing the internal configuration of the insertion section and the operation section of the endoscope according to the sixth embodiment; and
FIG. 14C is a schematic transverse cross-sectional view taken along a line 14C-14C in FIG. 14A and FIG. 14B.

### Brief Description of Embodiments

Modes for carrying out the present invention will now be described hereinafter with reference to the drawings.

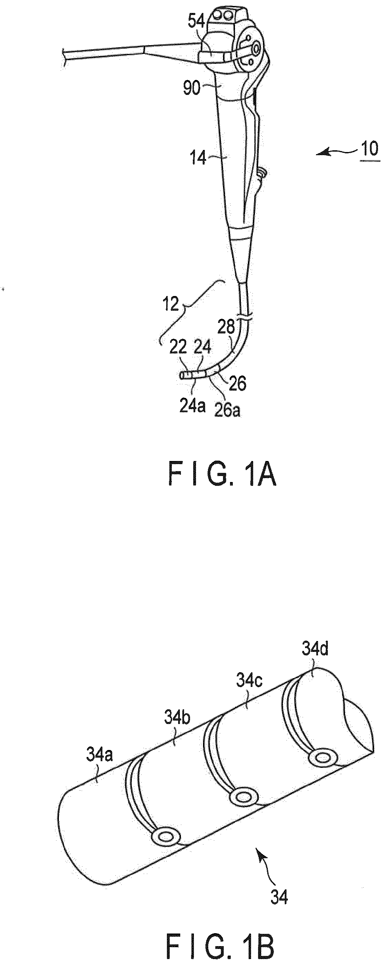

A first embodiment will now be explained with reference to FIG. 1A to FIG. 2C.

As shown in FIG. 1A, an endoscope 10 includes an elongated insertion section 12 and an operation section 14 provided at a proximal end portion of the insertion section 12. In the endoscope 10, an observation optical system and an illumination optical system which are not shown are arranged like a general endoscope. Further, in the endoscope 10, it is preferable to form a channel (not shown) which is used for supply air or supply water or into which a treatment tool is inserted.

The insertion section 12 includes a distal end hard portion 22, a first bending portion 24, a second bending portion 26, and a flexible tube portion (a tubular portion) 28 from a distal end side toward a proximal end side in the mentioned order. A proximal end of the flexible tube portion 28 is coupled with the operation section 14.

As will be described later, the first bending portion 24 functions as a so-called active bending portion that can be bent by operating the operation section 14. Furthermore, the second bending portion 26 functions as a so-called passive bending portion when a bending angle of the first bending portion 24 is small (a bending amount is small). The second bending portion 26 functions as the passive bending portion or exerts a function like a simple hard tubular body as a bending angle (a bending amount) of the first bending portion 24 increases.

The first bending portion 24 shown in FIG. 1A includes a first bending tube 34 in which bending pieces 34a, 34b, ... shown in FIG. 1B are aligned along an axial direction. The first bending tube 34 supports later-described first angle wires 60a and 60b to be movable in the axial direction thereof by using a non-illustrated wire guide provided to the respective bending pieces 34a, 34b, .... It is to be noted that a distal end of each of the first angle wires 60a and 60b is fixed to the most distal bending piece 34a of the first bending tube 34. The second bending portion 26 is formed like the bending tube 34 of the first bending portion 24 and includes a second bending tube 36 in which bending pieces (not shown) are aligned along the axial direction thereof. The second bending tube 36 supports later-described distal end-side traction members 78a and 78b by using a non-illustrated wire guide. It is to be noted that a distal end of each of the distal end-side traction members 78a and 78b is fixed to the most distal bending piece of the second bending tube 36.

The first bending tube 34 of the first bending portion 24 and the second bending tube 36 of the second bending tube 26 are bendable with respect to a common center axis C, and they can bend in two directions (an upward direction (a U direction) and a downward direction (a D direction)) from a state that each of the these tubes is straight (a bending angle is 0 degree) in this example. It is to be noted that lengths of the first and second bending tubes 34 and 36 can be appropriately set.

In this embodiment, although the description will be given as to the first bending portion 24 and the second bending portion 26 that can bend in the two directions, respectively, it is also preferable to provide a configuration in which the first bending portion 24 can bend in four directions and the second bending portion 26 can bend in the two directions or the first bending portion 24 can bend in the four directions and the second bending portion 26 can bend in the four directions.

In each of the first bending portion 24 and the second bending portion 26, for example, a braid (not shown) is arranged on the outer side of each of the first bending tube 34 and the second bending tube 36, and outer tube 24a and 26a which are made of, e.g., a rubber material and have elasticity are arranged on the outer side of the braid.

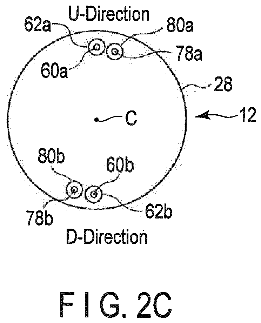

As shown in FIG. 2C, in the insertion section 12, a coil pipe 62a in which the later-described angle wire 60a is inserted is adjacent to a coil pipe 80a in which the distal end-side traction member 78a is inserted, and a coil pipe 62b in which the first angle wire 60b is inserted is adjacent to a coil pipe 80b in which the distal end-side traction member 78b is inserted. Further, the coil pipes 62b and 80b face the coil pipes 62a and 80a. In particular, it is preferable for the coil pipe 62b to face the center axis C of the flexible tube portion 28 with respect to the coil pipe 62a and for the coil pipe 80b to face the center axis C of the flexible tube portion 28 with respect to the coil pipe 80a.

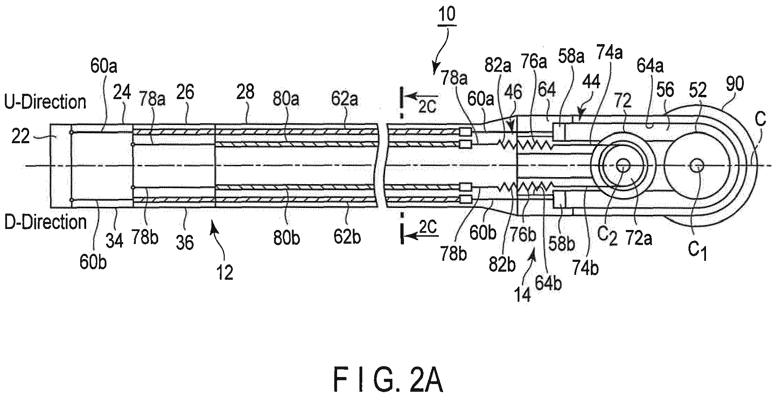

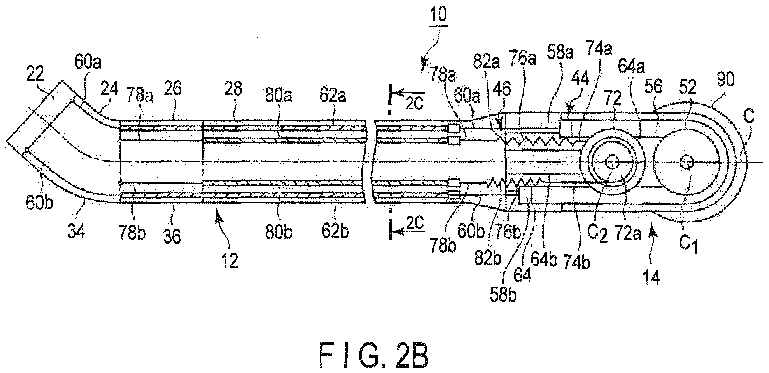

As shown in FIG. 2A and FIG. 2B, the endoscope 10 includes a first bending drive mechanism 44 configured to bend the first bending portion 24 in multiple directions and a second bending drive mechanism 46 that follows driving of the first bending drive mechanism 44 and is configured to maintain a straight state of the second bending portion 26 or bend the second bending portion 26 in the same direction as the bending direction of the first bending portion 24. That is, the first bending drive mechanism 44 and the second bending drive mechanism 46 are arranged in the insertion section 12 and the operation section 14.

The first bending drive mechanism 44 includes a first sprocket (a chain drum) 52 arranged in the operation section 14, a first bending portion operation knob (a bending operation input portion) 54, a chain 56 that is meshed with and wound around the first sprocket 52, first connection members 58a and 58b arranged at respective end portions of the chain 56, the first angle wires 60a and 60b, and the first coil pipes 62a and 62b in which the first angle wires 60a and 60b are inserted, respectively.

The operation section 14 includes a basal plate 64 therein. It is preferable for the basal plate 64 to have, e.g., a direction parallel to the axial direction of the insertion section 12 being longer than a direction orthogonal to the axial direction of the insertion section 12.

The first sprocket 52 is supported by the basal plate 64 and can rotationally move about a center axis C1 of the first sprocket 52. The first bending portion operation knob 54 is arranged outside the operation section 14 and can rotationally move the first sprocket 52 about the center axis C1. That is, the first sprocket 52 and the first bending portion operation knob 54 integrally move with respect to the operation section 14. Therefore, an operation amount of the first bending portion operation knob 54 is reflected on a moving length, i.e., an amount of rotational movement of the first sprocket 52. It is to be noted that, for example, a chain guide 64a is formed on the basal plate 64 so that the chain 56 can be moved in a predetermined direction when the first sprocket 52 is rotationally moved about the center axis C1. Further, the chain 56 can maintain a state that it is meshed with the first sprocket 52 and a later-described second sprocket 72.

A distal end of each of the first angle wires 60a and 60b is fixed at a proximal end of the distal end hard portion 22 that is also a distal end of the first bending tube 34 of the first bending portion 24. A proximal end of each of the first angle wires 60a and 60b is supported by each of the first connection members 58a and 58b. A distal end of each of the first coil pipes 62a and 62b is fixed at a proximal end of the bending tube 34 of the first bending portion 24 that corresponds to a position of a distal end of the bending tube 36 of the second bending portion 26. A proximal end of each of the first coil pipes 62a and 62b is supported, e.g., near a boundary between the proximal end portion of the insertion section 12 and the operation section 14. It is also preferable for the proximal end of each of the first coil pipes 62a and 62b to be fixed to the basal plate 64.

In this manner, the configuration that can bend the first bending portion 24 of the endoscope 10 according to this embodiment from the straight state in the U direction and the D direction which are opposite to each other (a bending mechanism of the first bending portion 24) is the same as a configuration of a general endoscope (a bending mechanism).

The second bending drive mechanism 46 has a configuration that interlocks with the first bending drive mechanism 44, can passively bend the second bending portion 26 in directions when external force is applied to the second bending portion even though the first bending portion 24 is straight, and generates compression force on a bending direction side of the first bending portion 24 with respect to the center axis C of the second bending tube 36 in the second bending portion 26 when a bending angle of the first bending portion 24 is increased from the straight state.

The second bending drive mechanism 46 includes the second sprocket (a chain drum) 72 which is arranged in the operation section 14 and includes a drum 72a integrally arranged thereon, a proximal end-side traction members (first traction members) 74a and 74b extended from the drum 72a, and buffer members 76a and 76b arranged at distal ends of the proximal end-side traction members 74a and 74b, respectively, distal end-side traction members (second traction members) 78a and 78b arranged at distal ends of the buffer portions 76a and 76b, respectively, and coil pipes 80a and 80b in which the distal end-side traction members 78a and 78b are inserted, respectively. That is, the buffer portions 76a and 76b are arranged between the proximal end-side traction members (the first traction members) 74a and 74b and the distal end-side traction members (the second traction members) 78a and 78b. It is to be noted that a guide portion 64b is formed on the basal plate 64 separately from the chain guide 64a. In the range of this guide portion 64b, the proximal end-side traction members 74a and 74b and the buffer portions 76a and 76b can move along a predetermined direction (the axial direction).

The second sprocket 72 may be supported to be rotationally movable about the center axis (a revolving axis) C2 thereof by the basal plate 64 arranged in the operation section 14, or may be supported to be rotationally movable about the center axis C2 thereof by, e.g., an extended portion (not shown) extended from the first sprocket 52, or may be supported by both the members. Further, since the chain 56 meshed with the first sprocket 52 is meshed with the second sprocket 72. Therefore, when the first bending portion operation knob 54 is rotationally moved about the center axis C1 thereof, the second sprocket 72 as well as the first sprocket 52 rotationally moves about the center axis C2 thereof in the same direction as the first sprocket 52. That is, the second bending drive mechanism 46 is driven in cooperation with the first bending drive mechanism 44. It is to be noted that an axis of the drum 72a is equal to an axis of the second sprocket 72 (the center axis C2), and a diameter of the drum 72a is smaller than a diameter of the second sprocket 72. Therefore, interference of the chain 56 and the proximal end-side traction members 74a and 74b or the buffer portions 76a and 76b can be avoided, and the outer envelope of the operation section 14 does not have to be enlarged.

The buffer portions 76a and 76b include elastic members 82a and 82b formed of, e.g., coil springs having stretching properties or made of a rubber material having stretching properties. In this embodiment, it is assumed that one coil spring is used as each of the elastic members 82a and 82b. The number of the elastic member 82a or 82b is not restricted to one with respect to each buffer portion 76a or 76b, and the plurality of elastic members may be used.

As will be described later, the elastic members 82a and 82b of the buffer portions 76a and 76b are adjusted to display its capability so that the second bending portion 26 hardly bends in the direction opposite to the bending direction of the first bending portion 24 when the first bending portion 24 is bent. For example, the elastic members 82a and 82b of the buffer portions 76a and 76b are adjusted in such a manner that, when the first bending portion 24 is bent at a maximum while the second bending portion 26 is straightened and no external force is applied to the second bending portion 26, the straight state of the second bending portion 26 is maintained without bending in the same direction as the bending direction of the first bending portion 24 and the second bending portion 26 is prevented from bending in a different direction, e.g., the opposite direction of the bending direction of the first bending portion 24 at the time of applying force in the different direction. It is to be noted that performing both the adjustment of the elastic members 82a and 82b and the adjustment of lengths of the distal end-side traction members 78a and 78b is also preferable.

Initial lengths of the elastic members (the coil springs) 82a and 82b of the buffer portions 76a and 76b slightly vary depending on a direction of the endoscope 10 or especially a direction of the operation section 14. When the insertion section 12 and the operation section 14 are turned sideways, the elastic members 82a and 82b of the buffer portions 76a and 76b have, e.g., natural lengths. When these sections are turned in the vertical direction (an up-and-down direction), the elastic members 82a and 82b of the buffer portions 76a and 76b are stretched by weights of the elastic members 82a and 82b themselves or gravity force of the distal end-side traction members 78a and 78b. Here, a state that the second bending portion 26 is straight irrespective of stretch of the elastic members 82a and 82b will be referred to as a neutral state.

A distal end of each of the distal end-side traction members 78a and 78b is fixed to the proximal end of the bending tube 34 of the first bending portion 24 that is also the distal end of the second bending tube 36 of the second bending portion 26. A proximal end of each of the distal end-side traction members 78a and 78b is fixed to each of the buffer portions 76a and 76b. That is, one end of each of the distal end-side traction members 78a and 78b is coupled with the second bending portion 26 and each of the distal end-side traction members 78a and 78b is extended toward the proximal end portion of the insertion section 12. A distal end of each of the second coil pipes 80a and 80b is fixed the proximal end of the bending tube 36 of the second bending portion 26 that corresponds to a position of the distal end of the flexible tube portion 28. A proximal end of each of the second coil pipes 80a and 80b is supported near, e.g., the boundary between the proximal end portion of the insertion section 12 and the operation section 14. It is also preferable for the proximal end of each of the second coil pipes 80a and 80b to be fixed to the basal plate 64.

It is to be noted that, since traction force applied to the proximal end-side traction members 74a and 74b and the distal end-side traction members 78a and 78b is lower than traction force applied to the first angle wires 60a and 60b, the proximal end-side traction members 74a and 74b and the distal end-side traction members 78a and 78b may be thinner than the first angle wires 60a and 60b. Further, the coil pipes 80a and 80b may be thinner than the coil pipes 62a and 62b.

The operation section 14 includes a cover 90. The cover 90 supports the basal plate 64, and covers the first sprocket 52, the second sprocket 72, the chain 56, the first connection members 58a and 58b, the first angle wires 60a and 60b, the proximal end-side traction members 74a and 74b, the buffer portions 76a and 76b, and the distal end traction members 78a and 78b which are arranged on the basal plate 64, and forms a grip portion gripped by, e.g., a left hand of a user of the endoscope 10. It is to be noted that the first bending portion operation knob 54 is provided outside the cover 90 and can be operated by, e.g., the left hand.

A function of the endoscope 10 according to this embodiment will now be described.

For example, in a state that the first bending portion 24 and the second bending portion 26 are straight, the first bending portion operation knob 54 is rotationally moved so that the first bending portion 24 can bend in the U direction. When the first sprocket 52 rotationally moves with the rotational movement of the first bending portion operation knob 54, one wire 60a of the first angle wires 60a and 60b is pulled through the chain 56 and the first connection members 58a and 58b. Since the distal end of the wire 60a is fixed to the bending piece 64a, when the wire 60a is pulled, the U direction side of the bending piece 34a is pulled toward the proximal end side, the bending pieces 34a, 34b, ... sequentially rotationally move, and the first bending tube 34 bends to toward the U direction side. Therefore, the first bending portion 24 bends in the U direction.

When the first sprocket 52 rotationally moves, the second sprocket 72 also rotationally moves in the same direction at the same time, and hence the drum 72a provided on the same axis (the center axis C2) as the second sprocket 72 integrally rotationally moves. Therefore, one proximal end-side traction member 74a of the proximal end-side traction members 74a and 74b is pulled. Therefore, the elastic member 82a of one buffer portion 76a of the buffer portions 76a and 76b stretches from, e.g., a state of the natural length or a stretched state because of its own weight or the like, i.e., the neutral state.

It is to be noted that the other proximal end-side traction member 74b, the other elastic member 82b of the buffer portion 76b, and the other distal end-side traction member 78b try to move to the distal end side of the insertion section 12, but these members deflect due to flexibility of the proximal end-side traction member 74b and the distal end-side traction member 78b themselves.

At the beginning of stretch of the elastic member 82a of the buffer portion 76a, a stretching amount of the elastic member 82a is small, and traction force for the distal end-side traction member 78a is weak. As the elastic member 82a of the buffer portion 76a stretches, the traction force becomes stronger in such a manner that the distal end side-traction member 78a is moved closer to the proximal end-side traction member 74a while the elastic member 82a of the buffer portion 76a stretches. Therefore, the tensile force is applied to the distal end-side traction member 78a of the distal end-side traction members 78a and 78b.

Here, since the elastic member 82a of the buffer portion 76a is arranged between the proximal end-side traction member 74a and the distal end-side traction member 78a, the tensile force can be reduced to be smaller than that in an example where the distal end-side traction member 78a is directly wound around the drum 72a and the distal end-side traction member 78a is pulled.

As described above, when the elastic member 82a of the buffer portion 76a is pulled by the proximal end-side traction member 74a, the tensile force for the distal end-side traction member 78a is weak at the beginning of stretch of the elastic member 82a. Further, as the elastic member 82a of the buffer portion 76a stretches, the traction force for the distal end-side traction member 78a gradually increases, and the buffer portion 76a can exercise the buffer function.

Furthermore, when the bending angle (a bending amount) of the first bending portion 24 in the U direction is increased from the state that the first bending portion 24 is straight (an initial state), since the tensile force is hardly applied to the distal end-side traction members 78a and 78b of the second bending drive mechanism 46 in the initial state, the second bending portion 26 functions as a passive bending portion that passively bends upon receiving external force. As the bending angle (the bending amount) of the first bending portion 24 is increased, the tensile force applied to the distal end-side traction member 78a gradually increases, but the second bending portion 26 functions as the passive bending portion like the initial state when the bending angle of the first bending portion 24 is small. When the bending angle of the first bending portion 24 is further increased, the tensile force applied to the distal end-side traction member 78a is further increased. Therefore, force is not immediately applied to the distal end-side traction member 78a between the distal end and the proximal end of the bending tube 36 of the second bending portion 26 when the first bending portion 24 is bent from the straight state, and compression force is applied to the bending direction side of the first bending portion with respect to the center axis C of the bending tube 36 of the second bending portion 26 as a bending amount of the first bending portion 24 increases.

It is to be noted that, in this embodiment, at the time of bending the first bending portion 24, even if the maximum tensile force is applied to the distal end-side traction member 78a, the compression force applied to the second bending portion 26 is applied in such a manner that the bending pieces of the bending tube 36 of the second bending portion 26 are not rotationally moved, i.e., that the second bending portion 26 can be maintained in a straight state. In other words, to achieve such a state, the elastic member 82a of the buffer portion 76a is selected and used, or adjustment of the elastic member 82a of the buffer portion 76a or adjustment of a length of the distal end-side traction member 78a is carried out.

Further, elastic force of the outer tube 26a, which is made of, e.g., a rubber material, of the bending tube 36 of the second bending portion 26 or stretch of the distal end-side traction member 78a can aid the second bending portion 26 to maintain the straight state.

In this manner, in a state that the first bending portion 24 has, e.g., the maximum bending angle, the compression force is applied to the distal end-side traction member 78a on the U direction side but no force is applied to the distal end-side traction member 78b on the D direction side between the distal end and the proximal end of the bending tube 36 of the second bending portion 26. Therefore, when external force is applied to the second bending portion 26 while the first bending portion 24 has, e.g., the maximum bending angle, the second bending portion 26 can readily bend in the U direction from the beginning due to the compression force of the distal end-side traction member 78a upon receiving pressing force from the D direction side, and hence the second bending portion 26 can bend in the U direction. As described above, when the second bending portion 26 bends in the same direction as the bending direction of the first bending portion 24, it is preferable for the second bending portion 26 to bend at a bending angle smaller than the bending angle of the first bending portion 24. On the other hand, when the pressing force is applied from the U direction side, the second bending portion 26 exerts resistance against bend in the D direction by the compression force applied to the second bending portion 26, and it tries maintaining the straight state.

It is to be noted that, after the first bending portion 24 is bent to the maximum bending amount, when the first bending portion operation knob 54 is operated and the first bending portion 24 is restored to the straight state, the second sprocket 72 rotationally moves in the same direction as the first sprocket 52. Therefore, the proximal end-side traction member 74a moves toward the distal end side of the insertion section 12, and a length of the elastic member 82a of the buffer portion 76a is returned to the neutral state. Therefore, when the bending angle of the first bending portion 24 becomes small, the compression force applied between the distal end and the proximal end of the second bending portion 26 by the distal end-side traction member 78a is eliminated.

In this embodiment, since the insertion section 12 and the operation section 14 of the endoscope 10 are symmetrically formed with respect to the center axis C, a description on a case where the first bending portion 24 is bent in the D direction will be omitted.

Therefore, the second bending portion 26 of the insertion section 12 of the endoscope 10 according to this embodiment functions as the passive bending portion when the first bending portion 24 is straight or has a small bending angle. Furthermore, as the bending angle of the first bending portion 24 increases, the second bending portion 26 functions as the passive bending portion that bends upon receiving external force in the same direction as the bending direction of the first bending portion 24, and it exerts resistance properties for avoiding bending or resistance properties for hardly bending upon receiving external force in a direction different from the bending direction of the first bending portion 24, e.g., the opposite direction. That is, the second bending portion 26 is automatically switched between a state as the passive bending portion and a state as a straight tubular body in accordance with a bending angle (a bending amount) and a bending direction of the first bending portion 24.

A situation where the insertion section 12 of this endoscope 10 is inserted into, e.g., the large intestine will now be roughly explained.

In case of inserting the distal end of the insertion section 12 into the large intestine from the anus side, an operator securely holds the insertion section 12 in his/her right hand, performs an operation for sending the insertion section 12 to the inner side of the large intestine or an operation for twisting the insertion section 12, senses, e.g., reaction force from the large intestine, and controls the endoscope 10 while considering a load on the large intestine.

When the distal end of the insertion section 12 has come near to a bend region, e.g., the sigmoid colon of the large intestine, the operator bends the first bending portion 24 and hooks it from the front side toward the inner side of the bend region. Although the second bending portion 26 can bend in the U direction due to external force while the first bending portion 24 is bent in, e.g., the U direction and hooked from the front side toward the inner side of the bend region, the second bending portion 26 is prevented from bending in the D direction.

Therefore, the first and second bending portions 24 and 26 of the insertion section 12 can be assuredly hooked from the front side toward the inner side of the bend region. At this time, when the second bending portion 26 bends from a state that the bend region is grabbed by the first bending portion 24, the current state changes to a state that the bend region is grabbed by the first and second bending portions 24 and 26. Therefore, the distal end of the insertion section 12 moves toward the inner side with respect to the bend region.

Moreover, the distal end of the insertion section 12 is fed toward the inner side of the large intestine, the first bending portion 24 is restored to the straight state from the bent state. Then, the distal end of the insertion section 12 can be moved from the bend region to the inner side.

As described above, according to this embodiment, the following effect can be obtained.

The second bending drive mechanism 46 coordinates with the first bending drive mechanism 44, can passively bend the second bending portion 26 in more than one direction while the first bending portion 24 is straight, and is configured to generate the compression force to the bending direction side of the first bending portion 24 with respect to the center axis C of the second bending tube 36 of the second bending portion 26 when the bending angle of the first bending portion 24 is increased from the straight state. Therefore, the second bending drive mechanism 46 can freely passively bend the second bending portion 26 when the first bending portion 24 is straight or nearly straight. Further, when the bending angle of the first bending portion 24 is increased by the first bending drive mechanism 44, the second bending drive mechanism 44 moves in response to this increase, and the second bending drive mechanism 46 can generate in the second bending portion 26 the compression force in the same direction as the bending direction of the first bending portion 24. Therefore, the second bending portion 26 can be restricted from bending in the opposite direction of the bending direction of the first bending portion 24, and the second bending portion 26 can maintain the straight state, or the second bending portion 26 can be aided to bend in the same direction as the bending direction of the first bending portion 24. That is, even if external force is received from, e.g., the U direction side of the second bending portion 26 while the first bending portion 24 is bent in the U direction, the second bending portion 26 can resist against this external force and can be prevented from bending in the D direction, and the second bending portion 26 can maintain the straight state or the nearly straight state. Furthermore, since the compression force is applied to the U direction side between the distal end and the proximal end of the second bending portion 26, when the external force is received from, e.g., the D direction side of the second bending portion 26, the second bending portion 26 easily bends to the U direction side. Therefore, according to this embodiment, for example, when the first bending portion 24 is bent in the U direction, the second bending portion 26 can readily bend in the U direction but can be prevented from bending in the D direction.

Moreover, since the second bending drive mechanism 46 has the buffer portions 76a and 76b, when motive power is transmitted from the first bending drive mechanism 44 to the second bending drive mechanism 46, timing for generating the compression force can be adjusted (delayed). For example, in a case where the elastic members 82a and 82b such as coil springs or rubber materials having elasticity are used for the buffer portions 76a and 76b, when the first bending portion 24 is bent by the first bending drive mechanism 44, the compression force can be generated on the bending side of the first bending portion 24 with respect to the center axis C in the second bending portion 26 at desired timing, and the compression force can be easily adjusted.

Additionally, since the buffer portion 76a is arranged between the proximal end-side traction member 74a and the distal end-side traction member 78a, the buffer member 76a can be arranged near the boundary between the insertion section 12 and the operation section 14, and hence the buffer portion 76a can be easily adjusted.

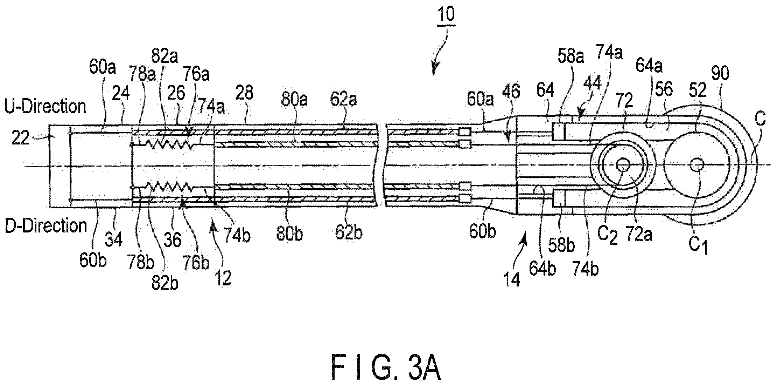

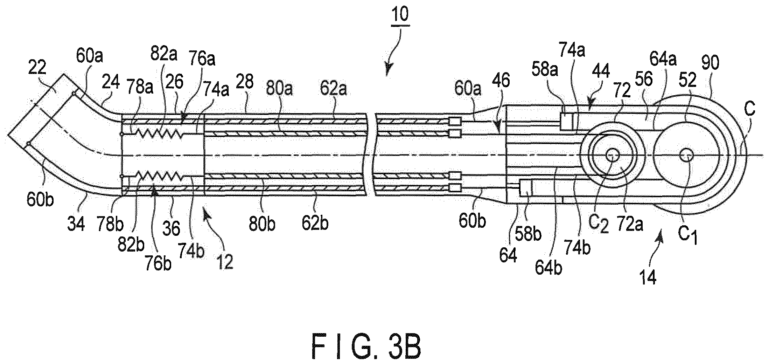

Each of FIG. 3A and FIG. 3B shows a modification of the endoscope 10 according to the first embodiment.

As shown in FIG. 3A and FIG. 3B, the positions of the buffer portions 76a and 76b are not restricted to the vicinity of the boundary between the proximal end portion of the insertion section 12 and the operation section 14. For example, the elastic members 82a and 82b of the buffer portions 76a and 76b may be arranged in the second bending portion 26. In this case, it is preferable to use, e.g., an elastic rubber material having elasticity rather than the coil springs for the elastic members 82a and 82b. Even such a configuration enables obtaining the same functions and effects as those of the first embodiment.

A second embodiment will now be described with reference to FIG. 4A to FIG. 4C. This embodiment is a modification of the first embodiment, like reference numerals denote the same members or members having the same functions as those explained in the first embodiment, and a detailed description will be omitted. Here, modifications of first connection members 58a and 58b, proximal ends of first angle wires 60a and 60b, and buffer portions 76a and 76b will be mainly described.

FIG. 4A shows a part of a first bending drive mechanism 44 and a second bending drive mechanism 46 on the U direction side arranged near a boundary between an insertion section 12 and an operation section 14. Although FIG. 4A to FIG. 4C show the U direction side alone, it is preferable for the D direction side to have the same configuration.

As shown in FIG. 4A, an engagement convex portion (an engagement portion) 92a is formed at a proximal end of the first angle wire 60a. In the first connection member 58a arranged at each end portion of a chain 56, engagement concave portions (engagement portions) 94a engaged with the engagement convex portion 92a of the first angle wire 60a are formed along the axial direction. Therefore, when a position of the engagement convex portion 92a is appropriately set with respect to the engagement concave portions 94a, initial traction force of the first angle wire 60a can be appropriately set.

As shown in FIG. 4A to FIG. 4C, the buffer portion 76a includes a proximal end-side coupling member 102a, an elastic member 82a, e.g., the coil spring described in the first embodiment, and a distal end-side coupling member 106a. The elastic member 82a is arranged between the proximal end-side coupling member 102a and the distal end-side coupling member 106a. An engagement convex portion (an engagement portion) 112a is formed at a distal end of the proximal end-side traction member 74a. An engagement concave portion (an engagement portion) 114a is formed at the proximal end-side coupling member 102a. Additionally, the engagement convex portion 112a is engaged with the engagement concave portion 114a and supported in such a manner that it does not come off during a regular operation.

The buffer portion 76a forms a slider mechanism 120a in cooperation with a proximal end of a distal end-side traction member 78a. This slider mechanism 120a includes a slider 122a fixed at the proximal end of the distal end-side traction member 78a and a slider receiving portion (a window portion) 124a that is formed in the distal end-side coupling member 106a and engaged with the slider 122a so that the slider 122a can slide in an axial direction of the distal end-side traction member 78a. That is, the slider 122a is fixed at the proximal end of the distal end-side traction member 78a. Furthermore, the distal end-side coupling member 106a includes the slider receiving portion (the window portion) 124a that is engaged with the slider 122a so that the slider 122a can slide in the axial direction of the distal end-side traction member 78a.

Therefore, the slider 122a can be relatively moved with respect to the slider receiving portion 124a. It is to be noted that the slider 122a can move in the axial direction with respect to the slider receiving portion 124a, but its movement is restricted along the direction deviating from the axial direction, and the slider 122a is supported in such a manner that it does not come off the slider receiving portion 124a during a regular operation.

Further, the slider 122a can move in the axial direction of the distal end-side traction member 78a with respect to the slider receiving portion 124a. If the elastic member 82a is in a neutral state that, e.g., the elastic member 82a has a natural length or is stretched by gravity force thereof or a state close to the neutral state, the slider 122a is apart from a distal end (a left side end portion in FIG. 4A) of the slider receiving portion 124a by, e.g., a length L. That is, looseness is formed between the slider 122a and the slider receiving portion 124a.

It is to be noted that a configuration of the buffer portion 76b is the same as a configuration of the buffer portion 76a, and hence a description thereof will be omitted.

A function of the endoscope 10 according to this embodiment will now be described.

At the time of bending a first bending portion 24, the proximal end-side traction member 74a is pulled toward the proximal end side. At this time, the elastic member 82a is moved to the proximal end side in the neutral state that, e.g., it has a natural length or is stretched by gravity force thereof or a state close to the neutral state.

At this time, since the looseness is present between the slider 122a and the slider receiving portion 124a, in a state that the first bending portion 24 has a small bending amount, applying external force enables bending a second bending portion 26 in both the U direction and the D direction.

As shown in FIG. 4B, the proximal end-side traction member 74a is pulled toward the proximal end side to bend the first bending portion 24 in the U direction. When the elastic member 82a is stretched, the distal end-side coupling member 106a is pulled toward the proximal end side. Therefore, the slider 122a comes into contact with the distal end of the slider receiving portion 124a. Until then, traction force is not applied to the distal end-side traction member 78a. That is, the slider mechanism 120a of the buffer member 76a is configured in such a manner that the looseness becomes maximum when the first bending portion 24 is straight and the looseness is gradually reduced as a bending angle of the first bending portion 24 increases from the straight state.

When the proximal end-side traction member 74a is further pulled toward the proximal end side from the state shown in FIG. 4B, the elastic member 82a is stretched, and the distal end-side coupling member 106a is pulled toward the proximal end side. Therefore, the tensile force is gradually applied to the distal end-side traction member 78a.

According to this embodiment, the compression force can be applied to the U direction side of the second bending portion 26 only in the range where, e.g., the bending angle of the first bending portion 24 is the U direction is large, and the second bending portion 26 can be prevented from bending in the D direction.

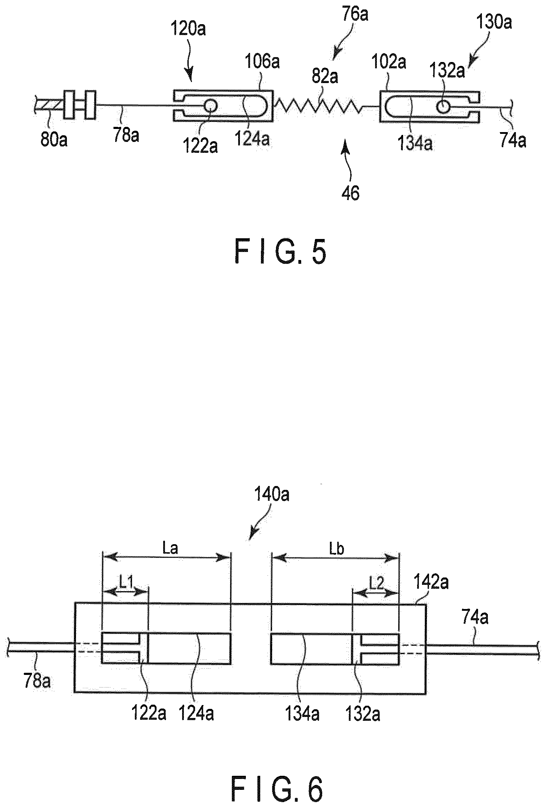

A third embodiment will now be described with reference to FIG. 5. This embodiment is a modification of the first and second embodiments, like reference numerals denote the same members or members having the same functions as those described in the first and second embodiments, and a detailed description thereof will be omitted. This embodiment is a further modification of the buffer portions 76a and 76b. Here, as shown in FIG. 5, there is provided an example where a configuration of a proximal end-side coupling member 102a is the same as a distal end-side coupling member 106a.

A buffer portion 76a forms a slider mechanism 130a in cooperation with a distal end of a proximal end-side traction member 74a. This slider mechanism 130a includes a slider 132a fixed at the distal end of the proximal end-side traction member 74a and a slider receiving portion (a window portion) 134a that is formed in a proximal end-side coupling member 102a and engaged with the slider 132a so that the slider 132a can slide in an axial direction of the proximal end-side traction member 74a. That is, at the distal end of the proximal end-side traction member 74a, the slider 132a having the same shape as, e.g., the slider 122a in the second embodiment is formed. The proximal end-side coupling member 102a includes a slider receiving portion (a window portion) 134a that allows the slider 132a of the proximal end-side traction member 74a to relatively move along its axial direction. This slider receiving portion 134a has the same shape as, e.g., the slider receiving portion 124a in the second embodiment.

It is to be noted that the slider 132a of the proximal end-side traction member 74a can move in the axial direction of the proximal end-side traction member 74a with respect to the slider receiving portion 134a, but it cannot move in a direction deviating from the axial direction. Further, the slider 132a is engaged with the slider receiving portion 134a and prevents from coming off during a regular operation.

As described above, the slider 132a can relatively move with respect to the slider receiving portion 134a on the proximal end side of the buffer portion 76a, the slider 122a can relatively move with respect to the slider receiving portion 124a on the distal end side of the buffer portion 76a, and hence looseness is formed until a second sprocket 72 rotationally moves about its center axis C2 to transmit force to a second bending portion 26.

A function of the endoscope 10 according to this embodiment will now be described hereinafter.

At the time of bending a first bending portion 24, the proximal end-side traction member 74a is pulled toward the proximal end side. At this time, the slider 132a comes into contact with the proximal end of the slider receiving portion 134a of the proximal end-side coupling member 102a. Further, an elastic member 82a moves to the proximal end side in a neutral state that, e.g., the elastic member 82a has a natural length or is stretched by gravity force thereof or a state close to the neutral state.

Therefore, the slider 122a comes into contact with the distal end of the slider receiving portion 124a of the distal end-side coupling member 106a. In a state that the slider 132a is in contact with the proximal end of the slider receiving portion 134a and the slider 122a is in contact with the distal end of the slider receiving portion 124a, when the proximal end-side traction member 74a is further pulled toward the proximal end side, the elastic member 82a is stretched.

Therefore, as described in the first and second embodiments, the tensile force is gradually applied to the distal end-side traction member 78a.

Therefore, since the play is present between the slider 122a and the slider receiving portion 124a and the play is also present between the slider 132a and the slider receiving portion 134a, when a bending amount of the first bending portion is small (including a straight state), the second bending portion 26 can bend in both the U direction and the D direction. On the other hand, when the bending amount of the first bending portion 24 increases and both the slider mechanisms 120a and 130a do not have the play, the compression force is gradually applied to the second bending portion 26.

That is, according to this embodiment, the compression force can be applied to the U direction side of the second bending portion 26 only in the range where, e.g., a bending angle of the first bending portion 24 in the U direction is large, and the second bending portion 26 can be prevented from bending in the D direction.

In the endoscope 10 according to this embodiment, the slider 122a at the proximal end of the distal end-side traction member 78a can slide with respect to the slider receiving portion 124a of the distal end-side coupling member 106a, the slider 132a at the proximal end of the proximal end-side traction member 74a can slide with respect to the slider receiving portion 134a of the proximal end-side coupling member 102a, and hence timing for applying the compression force to the second bending portion 26 when the first bending portion 24 is bent can be widely adjusted as compared with the case described in the second embodiment.

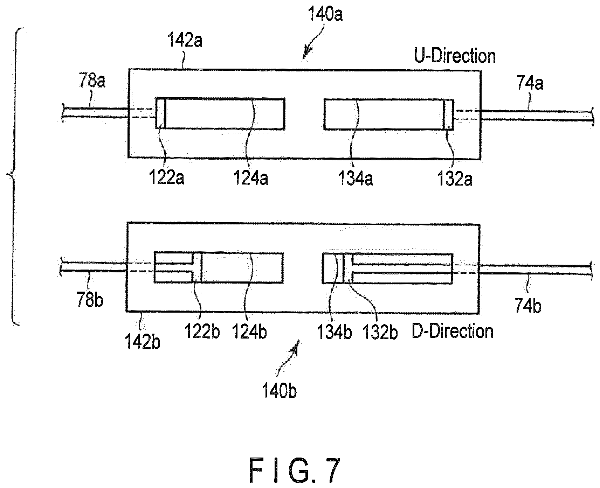

A fourth embodiment will now be described with reference to FIG. 6 and FIG. 7, this embodiment is a modification of the first to third embodiments, like reference numerals denote the same members or members having the same functions as those described in the first to third embodiments, and a detailed description will be omitted. A buffer portion 76a of an endoscope 10 according to this embodiment is an example that elastic members 82a and 82b, e.g., coil springs are eliminated from the configuration described in the third embodiment.

The buffer portion 76a forms a slider mechanism 140a in cooperation with a distal end of a proximal end-side traction member 74a and a distal end of a distal end-side traction member 78a. A buffer portion 76b forms a slider mechanism 140b in cooperation with a distal end of a proximal end-side traction member 74b and a distal end of a distal end-side traction member 78b. It is to be noted that, since the slider mechanisms 140a and 140b have the same configuration, one slider mechanism 140a will be mainly described.

As shown in FIG. 6, the slider mechanism 140a of the buffer portion 76a includes a frame member 142a in which two slider receiving portions 124a and 134a are aligned. The slider receiving portions 124a and 134a are preferably aligned along the longitudinal direction of an insertion section 12.

Here, it is determined that lengths of the slider receiving portions 124a and 134a in the longitudinal direction when the second bending portion 26 is straight are La and Lb. Further, it is also determined that a distance between a distal end of the slider receiving portion 124a and a slider 122a at the proximal end of the distal end-side traction member 78a is L1 and a distance between a proximal end of the slider receiving portion 134a and a slider 132a at the distal end of the proximal end-side traction member 74a is L2. It is to be noted that La is larger than L1 (≥0) and Lb is larger than L2 (≥0). At this time, (La+Lb)/2 is set larger than (L1+L2).

In a case where the first bending portion 24 is bent in the U direction, as shown in FIG. 7, when the first bending portion 24 has a predetermined bending angle, the slider 122a of the distal end-side traction member 78a comes into contact with the distal end of the slider receiving portion 124a of the frame member 142a, and the slider 132a of the proximal end-side traction member 74a comes into contact with the proximal end of the slider receiving portion 134a. Therefore, when the bending angle of the slider receiving portion 134a enters a predetermined state, the distal end-side traction member 78a is pulled. On the other hand, the slider 122b at the proximal end of the distal end-side traction member 78b is arranged at a position between the distal end and the proximal end of the slider receiving portion 124b of the frame member 142b, and the slider 132b at the distal end of the proximal end-side traction member 74b is arranged at a position between the distal end and the proximal end of the slider receiving portion 134b.

For example, in a state that the first bending portion 24 is bent in the U direction at a predetermined bending angle, when the length of the proximal end-side traction member 74a or the distal end-side traction member 78a or an axial width of the slider receiving portion 124a, 134a (the distance La, Lb) is set to provide the state depicted in FIG. 7, the second bending portion 26 functions as a passive bending portion in a process for gradually increasing the bending angle from the state that the first bending portion 24 is straight, and compression force can be applied to the U direction side of a bending tube 36 of the second bending portion 26 only in a state that the first bending portion 24 has the predetermined bending angle or a larger angle.

As described above, in this embodiment, an elastic member (e.g., a coil spring) is not provided. Therefore, a situation where the compression force is applied to the second bending portion 26 while the bending angle of the first bending portion 24 is large (a bending amount is large) (e.g., the first bending portion 24 has the maximum bending angle) is more preferable than the situation where the compression force is applied to the second bending portion 26 described in the second and third embodiments. With such a setting, the compression force is not applied to the second bending portion 26 when the bending angle of the first bending portion 24 is small, but the compression force can be applied to the second bending portion 26 when the first bending portion 24 has the predetermined bending angle or a higher angle.

It is to be noted that the description has been given as to the example where the two slider receiving portions 124a and 134a are formed in one frame member 142a in this embodiment, but both the slider 122a of the distal end-side traction member 78a and the slider 132a of the proximal end-side traction member 74a can be preferably movably arranged with respect to only one slider receiving portion in one frame member 142a. That is, it is also preferable to remove a partition between the slider receiving portions 124a and 134a of the frame member 142a.

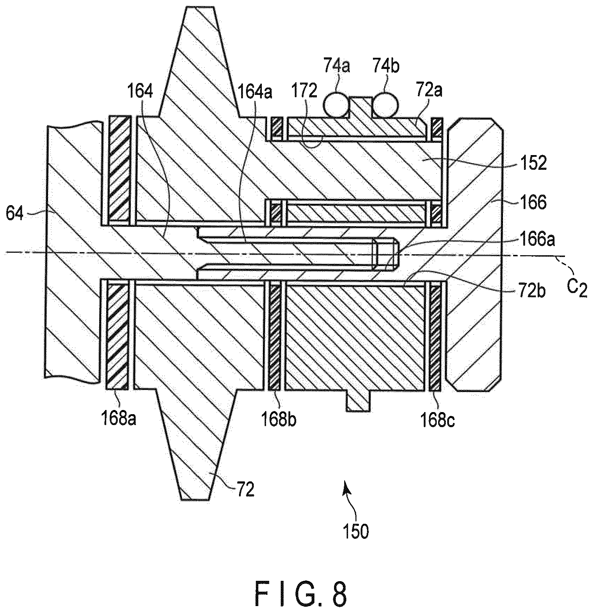

A fifth embodiment will now be described with reference to FIG. 8 to FIG. 11B. This embodiment is a modification of the first embodiment, like reference numerals denote the same members and members having the same functions as those explained in the first embodiment as much as possible, and a detailed description thereof will be omitted.

Although the example where the drum 72 is fixed to the second sprocket 72 in each of the first to fourth embodiments has been explained, a drum 72a according to this embodiment is separated from a second sprocket 72 as shown in FIG. 8. Further, in this embodiment, a description will be given as to an example where a buffer portion 150 (see FIG. 8) is formed of the second sprocket (a first revolving member) 72 and the drum (a second revolving member) 72a of a second bending drive mechanism 46. This embodiment is also a modification of the second to fourth embodiments, and the buffer portions 76a and 76b (see FIG. 4A to FIG. 7) explained in the second to fourth embodiments can be also used.

As shown in FIG. 8, the second sprocket 72 includes a shaft-like revolving force transfer portion (an arm) 152, which is formed at a position away from a revolving axis (a center axis) C2 on one opposite side of a surface facing a basal plate 64. The revolving axis C2 and the revolving force transfer portion 152 are formed to be substantially parallel to each other. Furthermore, the revolving force transfer portion (a movement portion) 152 moves while drawing an arc trajectory when the second sprocket 72 rotationally moves about the revolving axis C2 in tandem with the first sprocket 52.

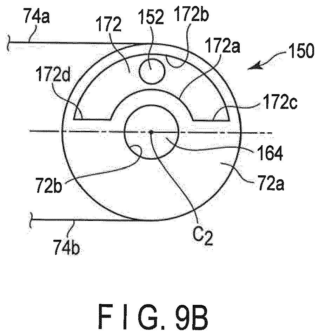

As shown in FIG. 9B, the drum 72a is formed into a substantially discoid shape. A circular through hole 72b in which a shaft 164 having the revolving axis (the center axis) C2 of the second sprocket 72 is arranged is formed at the center of the drum 72a. An inner diameter of the through hole 72b of the drum 72a is formed larger than an outer diameter of the shaft 164, and the shaft 164 can relatively smoothly revolve with respect to the drum 72a.

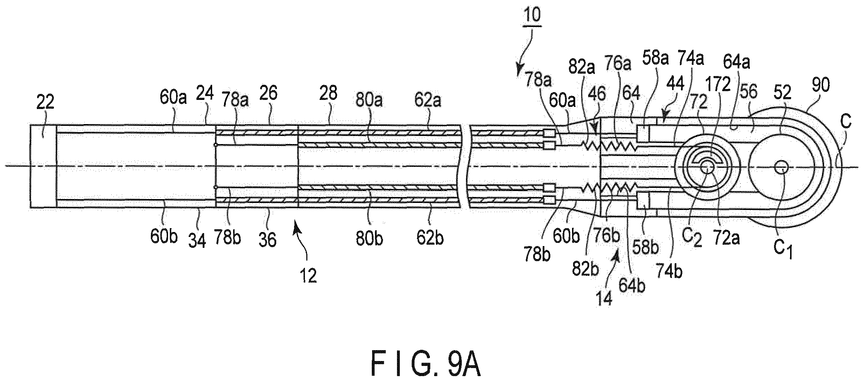

As shown in FIG. 9B, a groove portion 172 in which the revolving force transfer portion 152 is inserted and movably accommodated is formed in the drum 72a. The groove portion 172 of the drum 72a defines a region surrounded by an inner arch 172a, an outer arch 172b, and end portions 172c and 172d. It is to be noted that the inner arch 172a and the outer arch 172b are preferably formed as part of an arc with the center axis C2 at the center. It is to be noted that the groove portion 172 according to this embodiment is formed at a position close to the proximal end-side traction member 74a on the U direction side, but it can be also preferably formed at a position close to the proximal end-side traction member 74b on the D direction side.

When a first bending portion 24 is straight and the second sprocket 72 is in a state shown in FIG. 9A and the drum 72a is in the state shown in FIG. 9B, force (compression force) is not applied to a second bending portion 26, and the second bending portion 26 can passively bend in various directions with respect to the center axis C.

As shown in FIG. 8, a rotary member 166 that holds the second sprocket 72 and the drum 72a between itself and the basal plate 64 and has a substantially T-like longitudinal cross section is fixed to a shaft 164 erected in, e.g., a direction orthogonal to the basal plate 64. That is, the shaft 164 has, e.g., a male screw portion 164a, the rotary member 166 has a female screw portion 166a, and the second sprocket 72 and the drum 72a are accommodated between the basal plate 64 and the rotary member 166 when these screw portions 164a and 166a are screwed. It is to be noted that discoid slide plates 168a, 168b, and 168c are arranged between the basal plate 64 and the second sprocket 72, between the second sprocket 72 and the drum 72a, and between the drum 72a and the rotary member 166, respectively. Therefore, these slide plates 168a, 168b, and 168c can suppress occurrence of friction between these members and relatively slidably maintain these members.

As shown in FIG. 9B, the groove portion 172 of the drum 72a is formed around the center axis C2 in the range of, e.g., approximately 180 degrees or so. As shown in FIG. 9A and FIG. 9B, it is preferable to arrange the revolving force transfer portion 152 at the center between the end portions 172c and 172d of the groove portion 172 while maintaining the first bending portion 24 straight. At this time, it is preferable for the revolving force transfer portion 152 to be placed at a position that is the closest to a position where the proximal end-side traction member 74a on the U direction side moves away from the drum 72a (a contact point of the proximal end-side traction member 74a). Therefore, an operational feeling in case of bending the first bending portion 24 in the U direction can be the same as a counterpart in case of bending this portion in the D direction. It is to be noted that the drum 72a when the first bending portion 24 is maintained in the straight state is usually kept at a fixed position (e.g., a position at which the revolving force transfer portion 152 is close to the contact point of the drum 72a and the proximal end-side traction member 74a at a maximum) based on force equilibrium of the distal end-side traction members 78a and 78b, the buffer portions 76a and 76b, and the proximal end-side traction members 74a and 74b.

The range of the groove portion 172 is not restricted to substantially 180 degrees, and it can be appropriately set to, e.g., the range of substantially 30 degrees, the range of substantially 60 degrees, the range of substantially 90 degrees, or the range of substantially 120 degrees.

It is to be noted that the center axis C2 of the drum 72a and the revolving axis C2 of the second sprocket 72 is the same axis (the center axis C2), and an outer diameter of the drum 72a is smaller than an outer diameter of the second sprocket 72. Therefore, interference between a chain 56 and the proximal end-side traction members 74a and 74b or the buffer portions 76a and 76b can be avoided, and an outer envelope of an operation section 14 does not have to be increased in size.

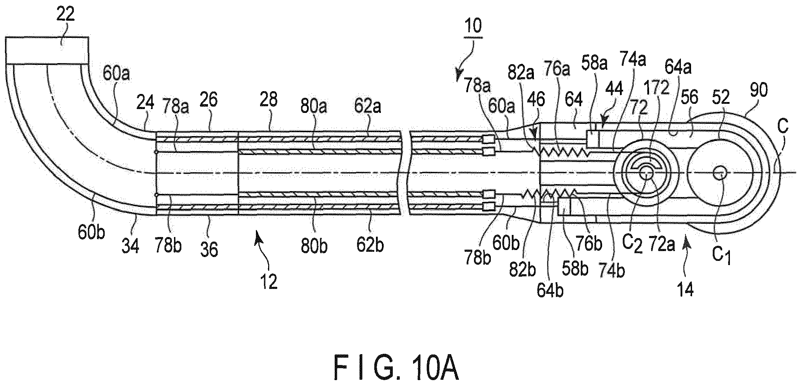

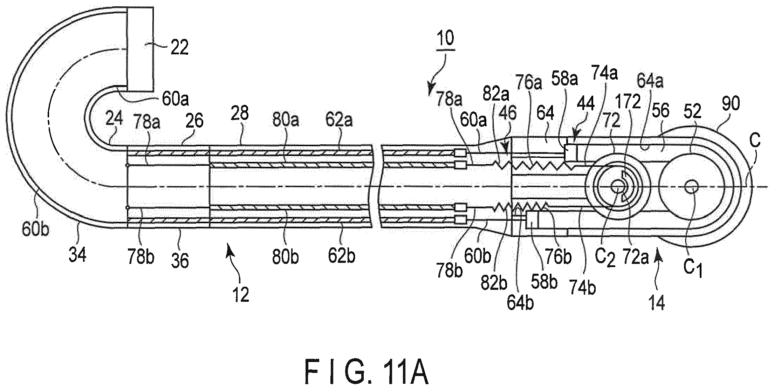

It is to be noted that the maximum bending angle of the first bending portion 24 is assumed to be 180 degrees (see FIG. 11A) in the U direction. That is, the bending angle of the first bending portion 24 is also allowed to be 90 degrees (see FIG. 10A) in the U direction as a matter of course. Further, the bending angle of the first bending portion 24 can be appropriately set with respect to a revolving angle of a knob 54, but the bending angle of the first bending portion 24 in this embodiment is assumed to be associated with the revolving angle of the drum 52 on one-on-one level. In this case, for example, a bending angle when the first bending portion 24 is present in a body cavity or the like can be easily grasped.

A function of the endoscope 10 according to this embodiment will now be described.

For example, as shown in FIG. 9A, when the first bending portion 24 and the second bending portion 26 are straight, the groove portion 172 of the drum 72a is close to the proximal end-side traction member 74a on the U direction side and away from the proximal end-side traction member 74b on the D direction side due to force equilibrium of the proximal end-side traction members 74a and 74b. That is, the groove portion 172 is close to the U direction side.

The first bending portion operation knob 54 is rotationally moved from the state shown in FIG. 9A so that the first bending portion 24 bends in the U direction, and the first bending portion 27 is bent in the U direction as shown in FIG. 10A and FIG. 11A.

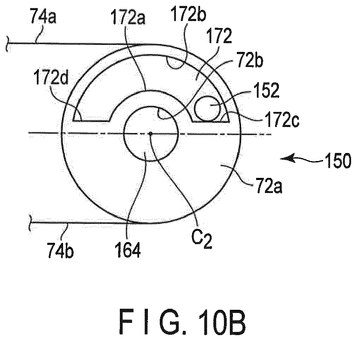

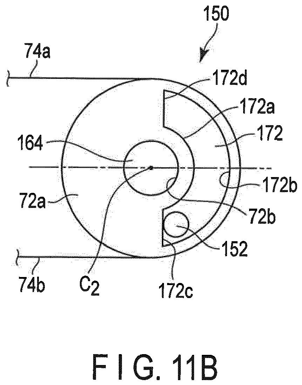

When the first sprocket 52 rotationally moves, the second sprocket 72 also rotationally moves in the same direction through the chain 56 at the same time. Therefore, the revolving power transfer portion 152 of the second sprocket 72 rotationally moves about the center axis C2 of the second sprocket 72. At this time, as shown in FIG. 10B, the revolving force transfer portion 152 comes into contact with the end portion 172c of the groove portion 172 of the drum 72a. Until then, the drum 72a tries maintaining its position, and force from the revolving force transfer portion 152 of the second sprocket 72 is not applied to the drum 72a. Furthermore, force is exerted to push the end portion 172c of the groove portion 172 by using the revolving force transfer portion 152 of the second sprocket 72 from the state shown in FIG. 10B. Therefore, as shown in FIG. 11A and FIG. 11B, the drum 72 rotationally moves about the rotary axis C2.

Since the proximal end-side traction members 74a and 74b are wound around the drum 72a, drive force is transmitted to the proximal end-side traction member 74a on the U direction side which is one of the proximal end-side traction members 74a and 74b by the revolving force transfer portion 152, the proximal end-side traction member 74a is pulled, and the elastic member 96a of the buffer portion 76a on the U direction side which is one of the buffer portions 76a and 76b stretches from, e.g., a state of a natural length or a state stretched by its own weight or the like, i.e., a neutral state.

At this time, the proximal end-side traction member 74b on the D direction side, the elastic member 82b of the buffer portion 76b, and the distal end-side traction member 76b try to move to the distal end side of the insertion section 12, but they deflect due to flexibility of the proximal end-side traction member 74b and the distal end-side traction member 78b themselves.

At the beginning of stretch of the elastic member 82a of the buffer portion 76a on the U direction side, a stretch amount of the elastic member 82a is small, and tensile force with respect to the distal end-side traction member 78a is weak. As the elastic member 82a of the buffer portion 76a stretches, tensile force increases so that the distal end-side traction member 78a can move close to the proximal end-side traction member 74a while the elastic member 82a of the buffer portion 76a stretches. Therefore, tensile force is applied to the distal end-side traction member 78a of the distal end-side traction members 78a and 78b.

That is, at the time of increasing the bending angle (the bending amount) in the U direction from the state that the first bending portion 24 is straight (an initial state), since almost no tensile force is applied to the distal end-side traction members 78a and 78b of the second bending drive mechanism 46 in the initial state, the second bending portion 26 functions as a passive bending portion that is passively bent upon receiving external force. As the bending angle (the bending amount) of the first bending portion 24 is increased, the tensile force applied to the distal end-side traction member 78a is gradually increased through the proximal end-side traction member 74a and the buffer portion 76a from the moment that the bending angle exceeds, e.g., substantially 90 degrees in this embodiment. At this time, if the bending angle of the first bending portion 24 is small (close to 90 degrees), the second bending portion 26 functions as the passive bending portion like the initial state. When the bending angle of the first bending portion 24 is further increased beyond substantially 90 degrees in this embodiment, the tensile force applied to the distal end-side traction member 78a further increases. Therefore, force is not immediately applied to the distal end-side traction member 78a between the distal end and the proximal end of the bending tube 36 of the second bending portion 26 at the time of bending the first bending portion 24 from the straight state, but compression force is applied to the bending direction side of the first bending portion 24 with respect to the center axis C of the bending tube 36 of the second bending portion 26 as the bending amount of the first bending portion 24 increases.

A description will now be given as to a case where the first bending portion 24 is returned to the neutral state from, e.g., the state that the first bending portion 24 is bent in the U direction at 180 degrees.

When the bending angle of the first bending portion 24 is reduced from the state shown in FIG. 11A to the state shown in FIG. 10A, the revolving power transfer portion 152 pushes the end portion 172d of the groove portion 172. Therefore, the drum 72a rotationally moves. At this time, no force is applied to the drum 72a itself by the revolving force transfer portion 152. Therefore, the drum 72a automatically rotationally moves by the elastic members 82a and 82b of the buffer portions 76a and 76b through the proximal end-side traction members 74a and 74b so that the force applied to the proximal end-side traction members 74a and 74b can be balanced. Therefore, the drum 72a returns to the position shown in each of FIG. 9A and FIG. 9B.

In this embodiment, since the insertion section 12 and the operation section 14 of the endoscope 10 are symmetrically formed with respect to the center axis C, a description on a situation where the first bending portion 24 is bent in the D direction will be omitted.

Therefore, the buffer portion 150 that has the revolving force transfer portion 152 of the second sprocket 72 and the groove portion 172 of the drum 72a can form looseness at the time of applying the compression force to the second bending portion 26. At this time, the looseness is maximum when the first bending portion 24 is straight, and the looseness is gradually reduced as the bending angle is increased from the state that the first bending portion 24 is straight. Therefore, the compression force is not applied to the second bending portion 26 when the bending angle of the first bending portion 24 is small, and the compression force applied to the second bending portion 26 can be gradually increased from the moment that the first bending portion 24 exceeds 90 degrees in this embodiment.

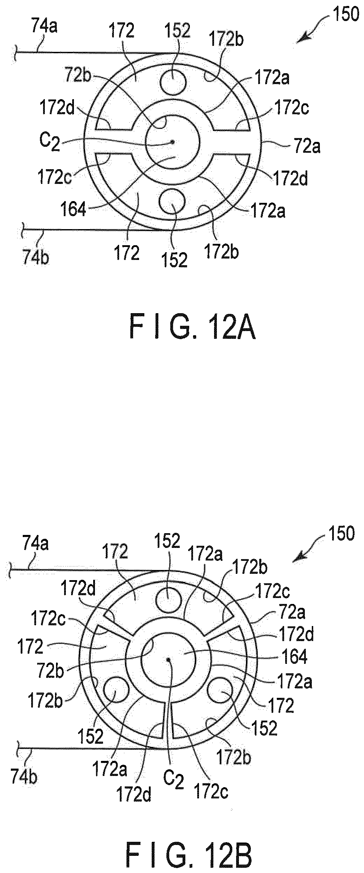

A modification of the fifth embodiment will now be briefly explained with reference to FIG. 12A and FIG. 12B.

A buffer portion 150 shown in FIG. 12A has two revolving force transfer portions 152 symmetrically formed on a second sprocket 72 with respect to a center axis C2 and two groove portions 172 symmetrically formed on a drum 72a with respect to the center axis C2. When the revolving force transfer portions 152 are arranged with respect to the two groove portions 172 in this manner, respectively, bias of gravity force applied to elastic members 82a and 82b of buffer portions 76a and 76b can be avoided.

The buffer portion 150 shown in FIG. 12B includes three revolving force transfer portions 152 symmetrically formed on a second sprocket 72 with respect to a center axis C2 and three groove portions 172 symmetrically formed on a drum 72a with respect to the center axis C2. This modification is an example that an amount of play is reduced as compared with the examples shown in FIG. 9A to FIG. 12A. As shown in FIG. 12B, each groove portion 172 according to this modification is formed to be narrower than the range of 120 degrees.

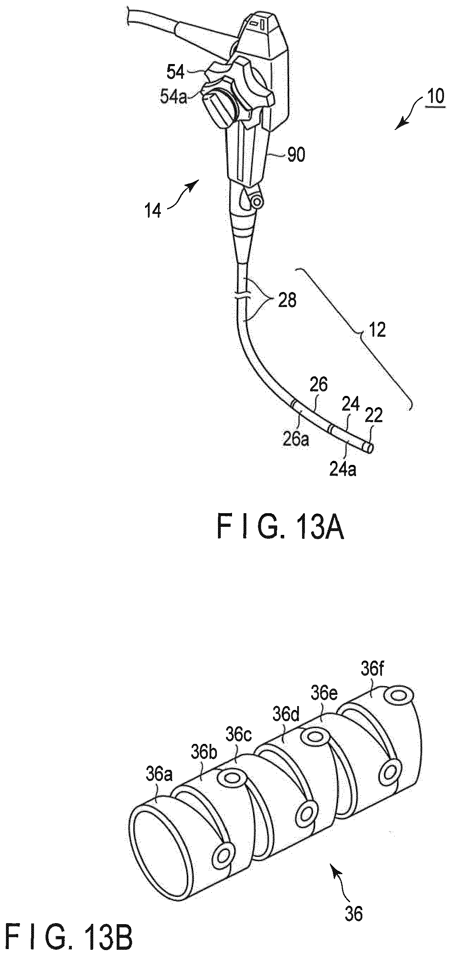

A sixth embodiment will now be described with reference to FIG. 13A to FIG. 14C. This embodiment is a modification of the first to fifth embodiments, like reference numerals denote the same members or members having the same functions as those explained in the first to fifth embodiments, and a detailed description thereof will be omitted.

A first bending portion 24 and a second bending portion 26 in an endoscope 10 according to this embodiment can bent in four directions (a U direction (a first direction), a D direction (a second direction), an R direction (a third direction), and an L direction (a fourth direction)) as shown in FIG. 13B, respectively.

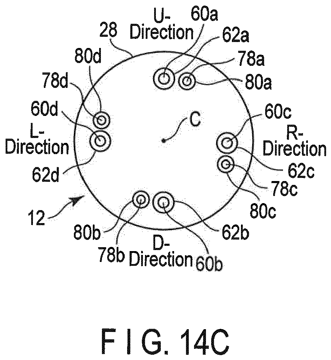

In this embodiment, a first bending drive mechanism 44 includes a configuration to bend the first bending portion 24 in the R direction and the L direction. In a cover 90 of an operation section 14, a different sprocket (not shown) that is arranged on the same axis as a first sprocket 52 and independently operates is arranged, and this different sprocket is coupled with a bending portion operation knob 54a arranged outside the operation section 14. A chain (not shown) is supported on the different sprocket, proximal ends of angle wires 60c and 60d shown in FIG. 14C are supported at end portions of the chain, and the angle wires 60c and 60d are inserted into coil pipes 62c and 62d. Therefore, the first bending portion 24 can bend in the four directions.

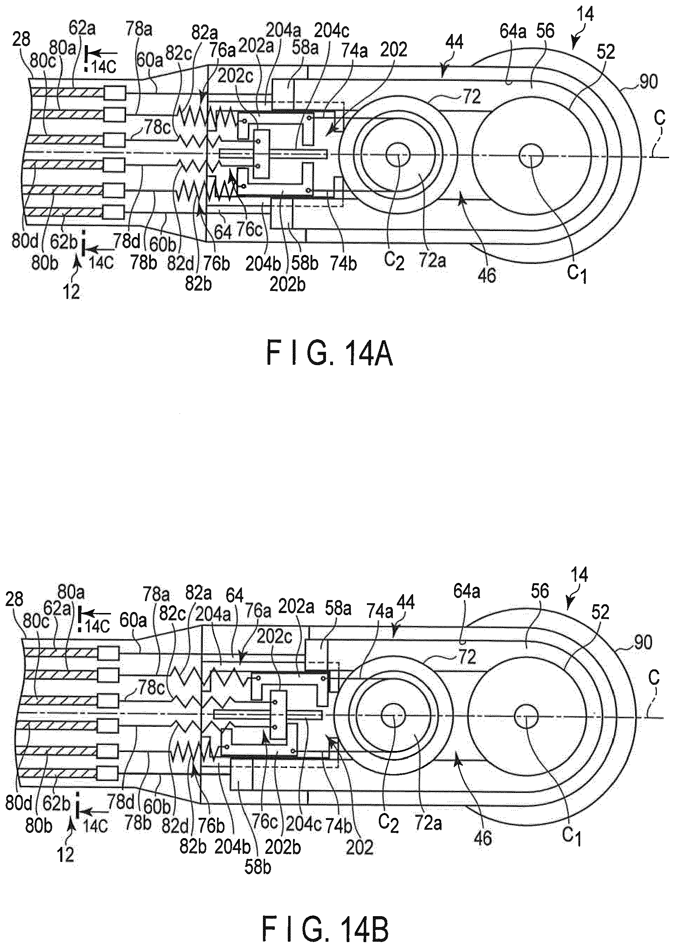

As shown in FIG. 14A and FIG. 14B, in this embodiment, a second bending drive mechanism 46 includes three buffer portions 76a, 76b, and 76c and an interlocking mechanism 202 that interlocks these buffer portions 76a, 76b, and 76c. The buffer portion 76a includes a first elastic member 82a and is coupled with a first interlocking member 202a of the interlocking mechanism 202. The buffer portion 76b includes a second elastic member 82b and is coupled with a second interlocking member 202b of the interlocking mechanism 202. The buffer portion 76c includes second and third elastic members 82c and 82d and is coupled with a third interlocking member 202c of the interlocking mechanism 202.

The first interlocking member 202a is arranged between a distal end of a proximal end-side traction member 74a and a proximal end of the elastic member 82a. The second interlocking member 202b is arranged between a distal end of a proximal end-side traction member 74b and a proximal end of the elastic member 82b. The third interlocking member 202c supports the proximal ends of the elastic members 82c and 82d.

A proximal end of a distal end-side traction member 78c is connected to a distal end of the elastic member 82c, and a proximal end of a distal end-side traction member 78d is connected to a distal end of the elastic member 82d. The distal end-side traction member 78c is inserted into a coil pipe 80c, and the distal end-side traction member 78d is inserted into a coil pipe 80d. Distal ends of the coil pipes 80c and 80d are fixed to a proximal end of a bending tube 36 of the second bending portion 26, and proximal ends of the same are supported at, e.g., a proximal end portion of the insertion section 12. Distal ends of the distal end-side traction members 78c and 78d are fixed between a proximal end of a bending tube 34 of the first bending portion 24 and a distal end of the bending tube 36 of the second bending portion 26.

Each of the first and second interlocking members 202a and 202b is formed to have, e.g., a substantially U-like longitudinal cross section and can move in a predetermined range on each of rails 204a and 204b of a basal plate 64 in a predetermined range along an axis parallel to a center axis C. The third interlocking member 202c is selectively engaged with the first and second interlocking members 202a and 202b and can move on a rail 204c of the basal plate 64 in a predetermined range along an axis parallel to the center axis C. That is, the first interlocking member 202a, the second interlocking member 202b, and the third interlocking member 202c can move in the predetermined ranges along the axial direction by the basal plate 64. Moreover, the third interlocking member 202c is configured to move from a given state to the proximal end side together with first interlocking member 202a or the second interlocking member 202b when the first interlocking member 202a or the second interlocking member 202b moves to the proximal end side.

In addition, as shown in FIG. 14C, the coil pipes 62a, 62b, 62c, and 62d and the angle wires 60a, 60b, 60c, and 60d in a flexible tube portion 28 are arranged in the U direction, the D direction, the R direction, and the L direction at intervals of substantially 90 degrees with respect to the center axis C. Likewise, the coil pipes 80a, 80b, 80c, and 80d and the distal end-side traction members 78a, 78b, 78c, and 78d are arranged in the U direction, the D direction, the R direction, and the L direction at intervals of substantially 90 degrees with respect to the center axis C.

A function of the endoscope 10 according to this embodiment will now be described.

Here, when the first bending portion 24 is bent in, e.g., the U direction from the state that the first bending portion 24 is straight, the second sprocket 72 rotationally moves, and a drum 72a also rotationally moves. Therefore, since the proximal end-side traction member 74a is pulled, the first interlocking member 202a is pulled toward the proximal end side, and the third interlocking member 202c is caught by the first interlocking member 202a and pulled toward the proximal end side.

Additionally, the elastic member 82a of the buffer portion 76a is pulled, and the elastic members 82c and 82d of the buffer portion 76c are pulled. Therefore, the elastic members 82a, 82c, and 82d stretch and gradually pull the distal end-side traction members 78a, 78c, and 78d. Therefore, in the second bending portion 26, compression force is applied to the bending direction side of the bending tube 34 of the first bending portion 24, and the compression force is applied to each side that is adjacent to the bending direction side of the second bending portion 26 at 90 degrees. That is, when the first bending portion 24 is bent to the U direction side, the compression force is applied to the U direction side, the R direction side, and the L direction side with respect to the center axis C of the second bending portion 26.

Here, in a state that external force is not applied to the second bending portion 26, the second bending portion 26 maintains a straight state. When the external force is applied to the second bending portion 26 from the U direction side of the first bending portion 24, the second bending portion 26 tries maintaining its straight state by using the compression force. At this time, the compression force prevents the second bending portion 26 from bending in the R direction or the L direction.

On the other hand, when the external force is applied from the D direction side, the second bending portion 26 bends in the U direction by this compression force.

It is to be noted that, in a state that the first bending portion 26 is bent at, e.g., a maximum bending angle, the second bending portion 26 is prevented from bending in the R direction and the L direction. That is because the compression force on the L direction side of the second bending portion 26 prevents the second bending portion 26 from bending in the R direction when the external force is applied from the L direction side of the second bending portion 26, and the compression force on the R direction side of the second bending portion 26 prevents the second bending portion 26 from bending in the L direction when the external force is applied from the R direction side of the second bending portion 26.

It is to be noted that, when the first interlocking member 202a is pulled toward the proximal end side by the proximal end-side traction member 74a, the third interlocking member 202c interlocks with the first interlocking member 202a and moves to the proximal end side. At this time, although the second interlocking member 202b moves in the opposite direction (the distal end side) of the first interlocking member 202a and the third interlocking member 202c, there is looseness since the proximal end-side traction member 74b has flexibility, and the second interlocking member 202b does not precipitously move to the distal end side.

Further, when bending the first bending portion 24 in the D direction is tried, the second interlocking member 202b is pulled toward the proximal end side by the proximal end-side traction member 74b. At this time, the third interlocking member 202c interlocks with the second interlocking member 202b and moves to the proximal end side. Although the first interlocking member 202a moves to the distal end side (the opposite side) of the second interlocking member 202b and the third interlocking member 202c, there is the play, and hence the first interlocking member 202a does not precipitously move to the distal end side.

In this embodiment, the example where the second bending drive mechanism 46 does not have a slider mechanism has been described, but the second bending drive mechanism 46 may have the slider mechanism.

It is to be noted that, in this specification and claims, the case where the bending portion is straight includes not only a state that the bending portion is actually straight (a state that the bending angle is 0 degree) but also a state that the bending angle is small (a substantially straight state). Furthermore, in this specification and claims, the same direction includes not only the actually same direction but also a slightly deviating direction.

### Reference Signs List

C...Center axis, C1...Center axis, C2...Center axis (Revolving axis), 10...Endoscope, 12...Insertion section, 14...Operation section, 22...Distal end hard portion, 24...First bending portion, 26...Second bending portion, 24a, 26a...Outer tube, 28...Flexible tube portion, 34...First bending tube, 36...Second bending tube, 44...First bending drive mechanism, 46...Second bending drive mechanism, 52...First sprocket, 54...First bending portion operation knob, 56...Chain, 58a, 58b...Connection members, 60a, 60b...angle wires, 62a, 62b...coil pipe, 64...basal plate, 64a...Chain guide, 64b...Guide portion, 72...Second sprocket, 72a...Drum, 74a, 74b...Proximal end-side traction members, 76a, 76b...Buffer portion, 78a, 78b...Distal end-side traction members, 80a, 80b...Coil pipe, 82a, 82b...Elastic members, 90...Cover.

## Claims

1. An endoscope (10) comprising:
a first bending portion (24) including a first bending tube (34) which is bendable in multiple directions (U, D) with respect to a straight state;
a second bending portion (26) which is provided on a proximal end side of the first bending portion (24) and includes a second bending tube (36) which is bendable in multiple directions (U, D) with respect to a straight state and also bendable in the same directions (U, D) as at least two directions of the first bending portion (24);
a first bending drive mechanism (44) which is configured to bend the first bending portion (24) in the multiple directions (U, D); and
a second bending drive mechanism (46) which is configured to interlock with the first bending drive mechanism (44), which is configured to bend the second bending portion (26) in multiple directions when external force is applied to the second bending portion (26) while the first bending portion (24) is straight, and is configured to generate compression force on a bending direction side of the first bending portion (24) with respect to a center axis (C) of the second bending tube (36) in the second bending portion (26) when a bending angle of the first bending portion (24) is increased with respect to the straight state, wherein the second bending drive mechanism (46) includes a buffer portion (76a, 76b) which is configured to buffer movement interlocked with the first bending drive mechanism (44),
**characterized in that** the buffer portion (76a, 76b) includes a slider mechanism (120), the slider mechanism includes a slider (122a) which is engaged with a slider receiving portion (124a) such that the slider can be relatively moved with respect to the slider receiving portion, wherein there is a looseness formed between the slider and the slider receiving portion, wherein said looseness is defined as a state wherein the slider is apart from a distal end of the slider receiving portion by a length L, the looseness being maximized when the first bending portion (24) is straight and the looseness being reduced as the bending angle of the first bending portion (24) increases from the straight state.

2. The endoscope according to claim 1, **characterized in that** the second bending drive mechanism (46) further includes:
a first traction member (74a, 74b) which is configured to interlock with the first bending drive mechanism (44); and
a second traction member (78a, 78b) which has one end coupled with the second bending portion (26) and is extended toward a proximal end portion of the insertion section (12), and
the buffer portion (76a, 76b) is arranged between the first traction member (74a, 74b) and the second traction member (78a, 78b).

3. The endoscope (10) according to claim 1, **characterized in that** the buffer portion (76a, 76b) has a stretchable elastic member (82a, 82b).

4. The endoscope (10) according to any one of claims 1 to 3, **characterized in that**
the second bending drive mechanism (46) includes:
a first revolving member (72) which is configured to interlock with the first bending drive mechanism (44) and is rotationally movable about a revolving axis (C2) of the first bending drive mechanism (44);
a second revolving member (72a) which is supported by the first revolving member (72) configured to be rotationally movable about a revolving axis (C2) of the revolving member (72); and
a traction member (78a, 78c, 78d) which is configured to interlock with the second revolving member (72a) and is configured to generate compression force in the second bending portion (26), and
the buffer portion includes:
a groove portion (172) which is provided around a revolving axis (C2) of the second revolving member (72a); and
a revolving force transfer portion (152) which is arranged in the groove portion (172) to be movable in the groove portion (172), which is configured to interlock with the first bending drive mechanism (44), which is configured to move along the groove portion (172) with respect to the second revolving member (72a), and which is configured to transmit revolving force to the second revolving member (72a).

5. The endoscope (10) according to any one of claims 1 to 4, **characterized in that**
the first bending tube (34) of the first bending portion (24) is bendable at least in a first direction (U) and a second direction (D) opposite to the first direction (U),
the second bending tube (36) of the second bending portion (26) is bendable in a third direction (R) and a fourth direction (L) opposite to the third direction (R) which are adjacent to the first direction (U) and the second direction (D) besides the first direction (U) and the second direction (D), and
the second bending drive mechanism (46) includes an interlocking mechanism (202) which is configured to generate the compression force in a first direction on a bending direction side of the first bending portion (24) with respect to the center axis (C) of the second bending tube (36) of the second bending portion (26), and a third direction and a fourth direction on the bending direction side which are adjacent to each other on the bending direction side when a bending angle of the first bending portion (24) is increased in the first direction from the straight state, and generates the compression force in a second direction on the bending direction side of the first bending portion (24) with respect to the center axis of the second bending tube (36) of the second bending portion (26), and the third direction and the fourth direction on the bending direction side which are adjacent to each other on the bending direction side when the bending angle of the first bending portion (24) is increased in the second direction from the straight state.

6. The endoscope (10) according to claim 5, **characterized in that** the interlocking mechanism (202) includes:
a first interlocking member (202a) and a second interlocking member (202b) which is configured to interlock with the first bending drive mechanism (44); and
a third interlocking member (202c) which is configured to interlock with the first interlocking member (202a) when the bending angle of the first bending portion (24) is increased in the first direction from the straight state, and which is configured to interlock with the second interlocking member (202b) when the bending angle of the first bending portion (24) is increased in the second direction from the straight state.

## Patentansprüche

1. Endoskop (10) aufweisend:
einen ersten Biegeabschnitt (24), der ein erstes Biegerohr (34) umfasst, das in mehrere Richtungen (U, D) in Bezug auf einen geraden Zustand biegbar ist;
einen zweiten Biegeabschnitt (26), der an einem Ende einer nahen Seite des ersten Biegeabschnitts (24) vorhanden ist und ein zweites Biegerohr (36) umfasst, das in mehrere Richtungen (U, D) in Bezug auf einen geraden Zustand biegbar ist und ebenso in dieselben Richtungen (U, D) wie die mindestens zwei Richtungen des ersten Biegeabschnitts (24) biegbar ist;
einen ersten Biegeantriebsmechanismus (44), der dazu eingerichtet ist, den ersten Biegeabschnitt (24) in die mehreren Richtungen (U, D) zu biegen; und
einen zweiten Biegeantriebsmechanismus (46), der dazu eingerichtet ist, mit dem ersten Biegeantriebsmechanismus (44) ineinander zu greifen, welcher dazu eingerichtet ist, den zweiten Biegeabschnitt (26) in mehrere Richtungen zu biegen, wenn eine äußere Kraft auf den zweiten Biegeabschnitt (26) aufgebracht wird, während der erste Biegeabschnitt (24) gerade ist, und dazu eingerichtet ist, eine Druckkraft auf eine Biegerichtungsseite des ersten Biegeabschnitts (24) in Bezug auf eine Mittelachse (C) des zweiten Biegerohrs (36) in dem zweiten Biegeabschnitt (26) zu erzeugen, wenn ein Biegewinkel des ersten Biegeabschnitts (24) in Bezug auf einen geraden Zustand erhöht wird, wobei der zweite Biegeantriebsmechanismus (46) einen Pufferabschnitt (76a, 76b) umfasst, der dazu eingerichtet ist, ein mit dem ersten Biegeantriebsmechanismus (44) ineinandergegriffene Bewegung zu puffern,
**dadurch gekennzeichnet, dass** der Pufferabschnitt (76a, 76b) einen Schiebemechanismus (120) umfasst, wobei der Schiebemechanismus einen Schieber (122a) umfasst, der mit einem Schieberaufnahmeabschnitt (124a) so im Eingriff ist, dass der Schieber relativ zu dem Schieberaufnahmeabschnitt bewegt werden kann, wobei ein Spiel zwischen dem Schieber und dem Schieberaufnahmeabschnitt vorhanden ist, wobei das Spiel als ein Zustand definiert ist, in welchem der Schieber um eine Länge L von einem fernen Ende des Schieberaufnahmeabschnitts entfernt ist, wobei das Spiel maximiert wird, wenn der erste Biegeabschnitt (24) gerade ist und das Spiel reduziert wird, sobald sich der Biegewinkel des ersten Biegeabschnitts (24) von dem geraden Zustand erhöht.

2. Endoskop nach Anspruch 1, **dadurch gekennzeichnet, dass** der zweite Biegeantriebsmechanismus (46) ferner umfasst:
ein erstes Traktionselement (74a, 74b), das dazu eingerichtet ist, mit dem ersten Biegeantriebsmechanismus (44) in Eingriff zu treten; und
ein zweites Traktionselement (78a, 78b), das ein mit dem zweiten Biegeabschnitt (26) gekoppeltes Ende hat und in Richtung eines nahen Endabschnitts des Einführbereichs (12) erstreckt, und
wobei der Pufferabschnitt (76a, 76b) zwischen dem ersten Traktionselement (74a, 74b) und dem zweiten Traktionselement (78a, 78b) angeordnet ist.

3. Endoskop (10) nach Anspruch 1, **dadurch gekennzeichnet, dass** der Pufferabschnitt (76a, 76b) ein dehnbares elastisches Element (82a, 82b) hat.

4. Endoskop (10) nach einem der Ansprüche 1 bis 3, **dadurch gekennzeichnet, dass**
der zweite Biegeantriebsmechanismus (46) umfasst:
ein erstes Drehelement (72), welches dazu eingerichtet ist, mit dem ersten Biegeantriebsmechanismus (44) in Eingriff zu treten und drehbar um eine Drehachse (C2) des ersten Biegeantriebsmechanismus (44) bewegbar ist;
ein zweites Drehelement (72a), welches mittels des ersten Drehelements (72) gestützt ist, das dazu eingerichtet ist, drehbar um eine Drehachse (C2) des Drehelements (72) bewegbar zu sein; und
ein Traktionselement (78a, 78c, 78d), welches dazu eingerichtet ist, mit dem zweiten Drehelement (72a) in Eingriff zu treten und dazu eingerichtet ist, eine Druckkraft in dem zweiten Biegeabschnitt (26) zu erzeugen, und
wobei der Pufferabschnitt umfasst:
einen Nutabschnitt (172), der um eine Drehachse (C2) des zweiten Drehelements (72a) vorhanden ist; und
einen Drehkraftübertragungsabschnitt (152), welcher in dem Nutabschnitt (172) angeordnet ist, um in dem Nutabschnitt (172) bewegbar zu sein, welcher dazu eingerichtet ist, mit dem ersten Biegeantriebsmechanismus (44) in Eingriff zu treten, welcher dazu eingerichtet ist, sich entlang des Nutabschnitts (172) in Bezug auf das zweite Drehelement (72a) zu bewegen, und welcher dazu eingerichtet ist, eine Drehkraft auf das zweite Drehelement (72a) zu übertragen.

5. Endoskop (10) nach einem der Ansprüche 1 bis 4, **dadurch gekennzeichnet, dass**
das erste Biegerohr (34) des ersten Biegeabschnitts (24) zumindest in einer ersten Richtung (U) und einer zweiten Richtung (D), entgegengesetzt der ersten Richtung (U), biegbar ist,
das zweite Biegerohr (36) des zweiten Biegeabschnitts (26) in eine dritte Richtung (R) und eine vierte Richtung (L), entgegengesetzt der dritten Richtung (R), biegbar ist, welche nahe der ersten Richtung (U) und der zweiten Richtung (D) neben der ersten Richtung (U) und der zweiten Richtung (D) sind, und
der zweite Biegeantriebsmechanismus (46) einen Eingriffsmechanismus (202) umfasst, welcher dazu eingerichtet ist, die Druckkraft in eine erste Richtung an einer Biegerichtungsseite des ersten Biegeabschnitts (24) in Bezug auf die Mittelachse (C) des zweiten Biegerohrs (36) des zweiten Biegeabschnitts (26) sowie in eine dritte Richtung und eine vierte Richtung an der Biegerichtungsseite zu erzeugen, welche nahe der Biegerichtungsseite sind, wenn ein Biegewinkel des ersten Biegeabschnitts (24) von dem geraden Zustand in die erste Richtung erhöht wird, und die Druckkraft in eine zweite Richtung an der Biegerichtungsseite des ersten Biegeabschnitts (24) in Bezug auf die Mittelachse des zweiten Biegerohrs (36) des zweiten Biegeabschnitts (26) erzeugt, und die dritte Richtung und die vierte Richtung an der Biegerichtungsseite, welche nah zueinander an der Biegerichtungsseite sind, wenn der Biegewinkel des ersten Biegeabschnitts (24) von dem geraden Zustand in die zweite Richtung erhöht wird.

6. Endoskop (10) nach Anspruch 5, **dadurch gekennzeichnet, dass** der Eingriffsmechanismus (202) umfasst:
ein erstes Eingriffselement (202a) und ein zweites Eingriffselement (202b), welches dazu eingerichtet ist, mit dem ersten Biegeantriebsmechanismus (44) in Eingriff zu treten; und
ein drittes Eingriffselement (202c), welches dazu eingerichtet ist, mit dem ersten Eingriffselement (202a) in Eingriff zu treten, wenn der Biegewinkel des ersten Biegeabschnitts (24) von dem geraden Zustand in die erste Richtung erhöht wird, und der dazu eingerichtet ist, mit dem zweiten Eingriffselement (202b) in Eingriff zu treten, wenn der Biegewinkel des ersten Biegeabschnitts (24) von dem geraden Zustand in die zweite Richtung erhöht wird.

## Revendications

1. Endoscope (10) comprenant :
une première partie de courbure (24) incluant un premier tube de courbure (34) qui peut être courbé dans de multiples directions (U, D) par rapport à un état rectiligne ;
une deuxième partie de courbure (26) qui est prévue sur un côté d'extrémité proximale de la première partie de courbure (24) et comprend un deuxième tube de courbure (36) qui peut être courbé dans de multiples directions (U, D) par rapport à un état rectiligne et pouvant également être courbé dans les mêmes directions (U, D) qu'au moins deux directions de la première partie de courbure (24) ;
un premier mécanisme d'entraînement de courbure (44) qui est configuré pour courber la première partie de courbure (24) dans les multiples directions (U, D) ; et
un deuxième mécanisme d'entraînement de courbure (46) qui est configuré pour s'interverrouiller avec le premier mécanisme d'entraînement de courbure (44), qui est configuré pour courber la deuxième partie de courbure (26) dans de multiples directions lorsqu'une force externe est appliquée à la deuxième partie de courbure (26) alors que la première partie de courbure (24) est rectiligne, et est configuré pour générer une force de compression sur un côté de direction de courbure de la première partie de courbure (24) par rapport à un axe central (C) du deuxième tube de courbure (36) dans la deuxième partie de courbure (26) lorsqu'un angle de courbure de la première partie de courbure (24) est augmenté par rapport à l'état rectiligne, dans lequel le deuxième mécanisme d'entraînement de courbure (46) comprend une partie tampon (76a, 76b) qui est configurée pour amortir le mouvement interverrouillé avec le premier mécanisme d'entraînement de courbure (44),
**caractérisé en ce que** la partie tampon (76a, 76b) comprend un mécanisme de coulissement (120), le mécanisme de coulissement comprend un élément coulissant (122a) qui est engagé avec une partie de réception (124a) d'élément coulissant d'une manière telle que l'élément coulissant peut être déplacé de manière relative par rapport à la partie de réception d'élément coulissant, dans lequel il existe un jeu formé entre l'élément coulissant et la partie de réception d'élément coulissant, dans lequel ledit jeu est défini comme un état dans lequel l'élément coulissant est espacé d'une extrémité distale de la partie de réception d'élément coulissant d'une longueur L, le jeu étant maximisé lorsque la première partie de courbure (24) est rectiligne et le jeu étant réduit à mesure que l'angle de courbure de la première partie de courbure (24) augmente par rapport à l'état rectiligne.

2. Endoscope selon la revendication 1, **caractérisé en ce que** le deuxième mécanisme d'entraînement de courbure (46) comprend en outre :
un premier élément de traction (74a, 74b) qui est configuré pour s'interverrouiller avec le premier mécanisme d'entraînement de courbure (44) ; et
un deuxième élément de traction (78a, 78b) qui comporte une extrémité couplée avec la deuxième partie de courbure (26) et s'étend vers une partie d'extrémité proximale de la section d'insertion (12), et
la partie tampon (76a, 76b) est agencée entre le premier élément de traction (74a, 74b) et le deuxième élément de traction (78a, 78b).

3. Endoscope (10) selon la revendication 1, **caractérisé en ce que** la partie tampon (76a, 76b) comporte un élément élastique (82a, 82b) étirable.

4. Endoscope (10) selon l'une quelconque des revendications 1 à 3, **caractérisé en ce que**
le deuxième mécanisme d'entraînement de courbure (46) comprend :
un premier élément rotatif (72) qui est configuré pour s'interverrouiller avec le premier mécanisme d'entraînement de courbure (44) et est mobile en rotation autour d'un axe de rotation (C2) du premier mécanisme d'entraînement de courbure (44) ;
un deuxième élément rotatif (72a) qui est supporté par le premier élément rotatif (72) configuré pour être mobile en rotation autour d'un axe de rotation (C2) de l'élément rotatif (72) ; et
un élément de traction (78a, 78c, 78d) qui est configuré pour s'interverrouiller avec le deuxième élément rotatif (72a) et est configuré pour générer une force de compression dans la deuxième partie de courbure (26), et
la partie tampon comprend :
une partie de rainure (172) qui est prévue autour d'un axe de rotation (C2) du deuxième élément rotatif (72a) ; et
une partie (152) de transfert de force de rotation qui est agencée dans la partie de rainure (172) pour être mobile dans la partie de rainure (172), qui est configurée pour s'interverrouiller avec le premier mécanisme d'entraînement de courbure (44), qui est configurée pour se déplacer le long de la partie de rainure (172) par rapport au deuxième élément rotatif (72a), et qui est configurée pour transmettre une force de rotation au deuxième élément rotatif (72a).

5. Endoscope (10) selon l'une quelconque des revendications 1 à 4, **caractérisé en ce que**
le premier tube de courbure (34) de la première partie de courbure (24) peut être courbé au moins dans une première direction (U) et une deuxième direction (D) opposée à la première direction (U),
le deuxième tube de courbure (36) de la deuxième partie de courbure (26) peut être courbé dans une troisième direction (R) et une quatrième direction (L) opposée à la troisième direction (R) qui sont adjacentes à la première direction (U) et à la deuxième direction (D) en plus de la première direction (U) et de la deuxième direction (D), et
le deuxième mécanisme d'entraînement de courbure (46) comprend un mécanisme d'interverrouillage (202) qui est configuré pour générer la force de compression dans une première direction sur un côté de direction de courbure de la première partie de courbure (24) par rapport à l'axe central (C) du deuxième tube de courbure (36) de la deuxième partie de courbure (26), et une troisième direction et une quatrième direction sur le côté direction de courbure qui sont adjacentes les unes aux autres sur le côté de direction de courbure lorsqu'un angle de courbure de la première partie de courbure (24) est augmenté dans la première direction par rapport à l'état rectiligne, et génère la force de compression dans une deuxième direction sur le côté de direction de courbure de la première partie de courbure (24) par rapport à l'axe central du deuxième tube de courbure (36) de la deuxième partie de courbure (26), et la troisième direction et la quatrième direction sur le côté de direction de courbure qui sont adjacentes les unes aux autres sur le côté de direction de courbure lorsque l'angle de courbure de la première partie de courbure (24) est augmenté dans la deuxième direction par rapport à l'état rectiligne.

6. Endoscope (10) selon la revendication 5, **caractérisé en ce que** le mécanisme d'interverrouillage (202) comprend :
un premier élément d'interverrouillage (202a) et un deuxième élément d'interverrouillage (202b) qui est configuré pour s'interverrouiller avec le premier mécanisme d'entraînement de courbure (44) ; et
un troisième élément d'interverrouillage (202c) qui est configuré pour s'interverrouiller avec le premier élément d'interverrouillage (202a) lorsque l'angle de courbure de la première partie de courbure (24) est augmenté dans la première direction par rapport à l'état rectiligne, et qui est configuré pour s'interverrouiller avec le deuxième élément d'interverrouillage (202b) lorsque l'angle de courbure de la première partie de courbure (24) est augmenté dans la deuxième direction par rapport à l'état rectiligne.
